# EUROPEAN PATENT APPLICATION

(11) **EP 2 687 850 A1**
(43) Date of publication of application: **22.01.2014**
(21) Application number: 12177051.5
(22) Date of filing: 19.07.2012
(51) Int. Cl.: G01N 33/68

(54) **Methods of stratifying for respiratory support therapy**

(71) Applicant: Roche Diagnostics GmbH, 68305 Mannheim (DE)
(72) Inventor: Boehm, Christine, 6331 Huenenberg (CH); Ehret, Christoph, Dr., 81477 München (DE); Klemt, Volker, 82362 Weilheim (DE); Roeddiger, Ralf, 69517 Gorxheimertal (DE); Pfeffer, Michael, 82377 Penzberg (DE)
(74) Representative: Schiweck, Weinzierl & Koch

(57) **Abstract**

The invention provides a method of (i) determining whether a new born subject is in need of respiratory support therapy, (ii) determining whether a foetus will be in need of respiratory support therapy after birth or (iii) stratifying a new born subject or a foetus for respiratory support therapy. The method comprises detecting the amount of pro-pulmonary surfactant-associated protein B (proSP-B), or an effective portion thereof, in a sample from the new born subject or foetus, or from amniotic fluid encompassing the foetus, and comparing the amount of proSP-B, or the effective portion thereof, in the sample to a threshold value. An increased amount of proSP-B, or the portion thereof indicates a need of the new born subject or of foetus after birth for respiratory support therapy.

SEQUENCE LISTING

<110>  Roche Diagnostics GmbH

<120>  METHODS OF STRATIFYING FOR RESPIRATORY SUPPORT THERAPY

<130>  ROC14325EP

<160>  9

<170>  PatentIn version 3.3

<210>  1
<211>  381
<212>  PRT
<213>  Homo sapiens

<400>  1

<210>  2
<211>  102
<212>  PRT
<213>  artificial sequence

<220>
<223>  peptide, amino acids 280-381 of proSP-B

<400>  2

Asp Asp Ser Ala Gly Pro Arg Ser Pro Thr Gly Glu Trp Leu Pro Arg
1               5                   10                  15

Asp Ser Glu Cys His Leu Cys Met Ser Val Thr Thr Gln Ala Gly Asn
            20                  25                  30

Ser Ser Glu Gln Ala Ile Pro Gln Ala Met Leu Gln Ala Cys Val Gly
        35                  40                  45

Ser Trp Leu Asp Arg Glu Lys Cys Lys Gln Phe Val Glu Gln His Thr
    50                  55                  60

Pro Gln Leu Leu Thr Leu Val Pro Arg Gly Trp Asp Ala His Thr Thr
65                  70                  75                  80

Cys Gln Ala Leu Gly Val Cys Gly Thr Met Ser Ser Pro Leu Gln Cys
                85                  90                  95

Ile His Ser Pro Asp Leu
            100

<210>  3
<211>  76
<212>  PRT
<213>  artificial sequence

<220>
<223>  peptide, amino acids 295-370 of proSP-B

<400>  3

Arg Asp Ser Glu Cys His Leu Cys Met Ser Val Thr Thr Gln Ala Gly
1               5                   10                  15

Asn Ser Ser Glu Gln Ala Ile Pro Gln Ala Met Leu Gln Ala Cys Val
            20                  25                  30

Gly Ser Trp Leu Asp Arg Glu Lys Cys Lys Gln Phe Val Glu Gln His
        35                  40                  45

Thr Pro Gln Leu Leu Thr Leu Val Pro Arg Gly Trp Asp Ala His Thr
    50                  55                  60

Thr Cys Gln Ala Leu Gly Val Cys Gly Thr Met Ser
65                  70                  75

<210>  4
<211>  78
<212>  PRT
<213>  artificial sequence

<220>
<223>  peptide, amino acids 204-281 of proSP-B

<400>  4

Pro Leu Pro Tyr Cys Trp Leu Cys Arg Ala Leu Ile Lys Arg Ile Gln

1               5                   10                  15

Ala Met Ile Pro Lys Gly Ala Leu Ala Val Ala Val Ala Gln Val Cys
            20                  25                  30

Arg Val Val Pro Leu Val Ala Gly Gly Ile Cys Gln Cys Leu Ala Glu
        35                  40                  45

Arg Tyr Ser Val Ile Leu Leu Asp Thr Leu Leu Gly Arg Met Leu Pro
    50                  55                  60

Gln Leu Val Cys Arg Leu Val Leu Arg Cys Ser Met Asp Asp
65                  70                  75

<210>  5
<211>  103
<212>  PRT
<213>  artificial sequence

<220>
<223>  peptide, amino acids 279 to 381 of proSP-B

<400>  5

Met Asp Asp Ser Ala Gly Pro Arg Ser Pro Thr Gly Glu Trp Leu Pro
1               5                   10                  15

Arg Asp Ser Glu Cys His Leu Cys Met Ser Val Thr Thr Gln Ala Gly
            20                  25                  30

Asn Ser Ser Glu Gln Ala Ile Pro Gln Ala Met Leu Gln Ala Cys Val
        35                  40                  45

Gly Ser Trp Leu Asp Arg Glu Lys Cys Lys Gln Phe Val Glu Gln His
    50                  55                  60

Thr Pro Gln Leu Leu Thr Leu Val Pro Arg Gly Trp Asp Ala His Thr
65                  70                  75                  80

Thr Cys Gln Ala Leu Gly Val Cys Gly Thr Met Ser Ser Pro Leu Gln
                85                  90                  95

Cys Ile His Ser Pro Asp Leu
            100

<210>  6
<211>  10
<212>  PRT
<213>  artificial sequence

<220>
<223>  peptide, amino acids 160 to 169 of proSP-B

<400>  6

Leu Pro Lys Pro Leu Arg Asp Pro Leu Pro
1               5                   10


<210>  7
<211>  12
<212>  PRT
<213>  artificial sequence

<220>
<223>  peptide, amino acids 323 to 334 of proSP-B

<400>  7

Gln Ala Cys Val Gly Ser Trp Leu Asp Arg Glu Lys
1               5                   10


<210>  8
<211>  10
<212>  PRT
<213>  artificial sequence

<220>
<223>  peptide, amino acids 285 to 294 of proSP-B

<400>  8

Pro Arg Ser Pro Thr Gly Glu Trp Leu Pro
1               5                   10


<210>  9
<211>  50
<212>  PRT
<213>  artificial sequence

<220>
<223>  peptide, amino acids 285 to 334 of proSP-B

<400>  9

Pro Arg Ser Pro Thr Gly Glu Trp Leu Pro Arg Asp Ser Glu Cys His
1               5                   10                  15


Leu Cys Met Ser Val Thr Thr Gln Ala Gly Asn Ser Ser Glu Gln Ala
            20                  25                  30


Ile Pro Gln Ala Met Leu Gln Ala Cys Val Gly Ser Trp Leu Asp Arg
        35                  40                  45


Glu Lys
    50

## Description

### FIELD OF THE INVENTION

The present invention relates to methods of stratifying for respiratory support therapy, in particular for assessing whether respiratory support therapy is or will be needed. In more detail, the invention provides methods for determining whether a new born subject is in need of respiratory support therapy or whether a foetus will be in need of respiratory support therapy after birth.

### BACKGROUND OF THE INVENTION

Lung surfactant is a mixture of lipids, mostly phospholipids (approx. 80%), neutral lipids (10%) and so-called surfactant proteins (10%). The latter are essential for normal breathing due to their ability to reduce alveolar surface tension and thereby preventing alveolar collapse during expiration. There are four surfactant proteins (SP-A, SP-B, SP-C and SP-D) with the hydrophobic surfactant proteins SP-B and SP-C being particularly important in maintaining the surface active film in the alveolus. The other two and hydrophilic surfactant protein SP-A and SP-D have a role in host defense and pathogen clearance and inflammatory responses The surfactant is synthesized and secreted into the alveolar fluid by alveolar type II cells (Andreeva, A.V., et al., Am J Physiol Lung Cell Mol Physiol (2007) 293, L259-L271).

SP-B is the most important surfactant in maintaining the alveolar surface pressure as a severe SP-B deficiency (i.e. a 75% reduction in SP-B content in the airspaces) results in fatal respiratory distress syndrome (RDS).

Respiratory distress syndrome in the newborn can be due to inherited disorders of surfactant metabolism by mutations in the genes encoding surfactant proteins-B or -C (SFTPB, SFTPC) (Hamvas, A., Chinese Medical Journal (2010) 123, 20, 2943-2947), but frequently occurs in preterm newborns with immaturity of the respiratory system and is a major cause of mortality and morbidity of preterm newborns. RDS commences at or shortly after birth and increases in severity over the first 2 days of life. If left untreated death can occur from progressive hypoxia and respiratory failure. The early respiratory distress is characterized by cyanosis, grunting, retraction, and tachypnea. Respiratory failure may develop and is indicated by blood gas analysis. The diagnosis can be confirmed on chest X-ray with a classical "ground glass" appearance and air bronchograms (Engle, W.A., PEDIATRICS (2008) 121, 2, 419-432).

The Vermont Oxford Neonatal Network definition requires that babies have a PaO₂ <50 mm Hg (<6.6 kPa) in room air, central cyanosis in room air or need for supplemental oxygen to maintain PaO₂) >50 mm Hg (>6.6 kPa) as well as classical chest X-ray appearances (Engle, 2008, supra).

Common treatments for RDS include supplemental oxygen and nasal continuous positive airway pressure (NCPAP; by varying the flow rate or providing a constant flow and varying Pressure). For severe RDS, surfactant administration during intubation and mechanical ventilation is used (de Winter, J.P., et al., Eur J Pediatr (2010) 169, 777-782; Stevens, T.P., et al., The Cochrane Library (2008), Issue 3).

Treating newborns in respiratory distress with surfactant improves clinical outcomes as the surfactant reduces initial inspired oxygen and ventilation requirements and improves the outcome. However, an ongoing debate when to start respiratory support therapy such as surfactant replacement therapy. In a preventive approach surfactant is administered to infants at high risk of developing respiratory distress syndrome before the onset of respiratory symptoms or after initial resuscitation within 10 to 30 minutes and up to 2 hours after birth. In a treatment strategy surfactant is given to preterm infants with established respiratory distress syndrome. Surfactant is then most often administered within the first 12 hours after birth when specified threshold criteria for respiratory distress syndrome are met. (Engle, 2008, supra)

Hence, there exists a need for means to assess whether a newborn is or will be in need of respiratory support therapy. Accordingly it is an object of the present invention to provide a method that allows assessing such need of respiratory support therapy. This object is solved by the method of claim 1.

### SUMMARY OF THE INVENTION

In some aspects the present disclosure generally relates to the determination whether a newborn or a foetus has an immature lung. In particular, the present invention can be taken to provide a method for assessing the occurrence or the susceptibility to a condition associated with a respiratory disorder and a method of respiratory disorder risk stratification. The methods and uses provided by the present invention are based on employing proSP-B, or an effective portion of proSP-B (see below), as a biomarker for identifying a respiratory disorder or identifying a predisposition of a newborn or a foetus of developing a respiratory disorder. In the context of the present invention proSP-B levels may be determined using any desired technique. In some embodiments means may be employed that indirectly indicate proSP-B levels.

The methods and uses of the present invention are generally carried out *in vitro.* Each of the methods and uses defined below may consist of the step or steps described. Each of the methods and uses defined below may include the indicated steps and in addition include further steps. Samples from a patient, which may also be referred to as test samples, on which a method or use defined herein is carried out may have been obtained by a physician. The methods and uses defined in the following may be carried using any suitable equipment in any desired setting such as a laboratory. Typically results of the method will be reviewed or analysed by a physician for diagnostic purposes.

According to a first aspect, the invention provides a method of determining whether a new born subject is in need of respiratory support therapy. The method typically includes detecting the amount of pro-pulmonary surfactant-associated protein B (proSP-B), or an effective portion thereof, in a sample from the new born subject. The method also typically includes comparing the detected amount of proSP-B, or the effective portion, in the sample to a threshold value. Further, the method typically includes determining that the new born subject is in need of respiratory support therapy. The need of the new born subject of respiratory support therapy is determined if the amount of proSP-B, or the portion thereof, relative to a threshold value is increased. It is determined that the new born subject is not in need of respiratory support therapy if the amount of proSP-B, or the portion thereof, relative to a threshold value is not increased or below the threshold value.

The method according to the first aspect generally includes providing a sample from the new born subject. The method further generally includes comparing the amount of proSP-B, or the effective portion, in the sample to a threshold value.

According to a particular embodiment of the method according to the first aspect, the amount of proSP-B, or of an effective portion thereof, is monitored at certain, e.g. predetermined, time intervals. Samples from the subject may be provided that have been obtained at the corresponding time points.

According to a second aspect, the invention provides a method of determining whether a foetus will be in need of respiratory support therapy after birth. The method typically includes detecting the amount of proSP-B, or an effective portion thereof, in a sample from the foetus or from amniotic fluid encompassing the foetus. Further the method typically includes comparing the detected amount of proSP-B, or of the effective portion thereof, in the sample to a threshold value. The method typically also includes determining that the foetus will be in need of respiratory support therapy after birth if the amount of proSP-B, or the portion thereof, relative to a threshold value is increased. The method typically also includes determining that the new born subject is not in need of respiratory support therapy if the amount of proSP-B, or the portion thereof, relative to a threshold value is not increased or below the threshold value.

The method according to the second aspect generally includes providing a sample from the foetus, or from amniotic fluid encompassing the foetus. According to a particular embodiment of the method according to the second aspect, the amount of proSP-B, or an effective portion thereof, is monitored at certain, e.g. predetermined, time intervals. Samples from the subject may be provided that have been obtained at the corresponding time points.

According to a third aspect, the invention provides a method of stratifying a new born subject or a foetus for respiratory support therapy. The method typically includes detecting the amount of proSP-B, or an effective portion thereof, in a sample from the new born subject, the foetus or from amniotic fluid encompassing the foetus. Typically the method also includes comparing the detected amount of proSP-B, or the effective portion, in the sample to a threshold value. Further, the method typically includes stratifying the new born subject or the foetus for respiratory support therapy if the amount of proSP-B, or the portion thereof, relative to a threshold value is increased. The method typically also includes not stratifying the new born subject or the foetus for respiratory support therapy if the amount of proSP-B, or the portion thereof, relative to a threshold value is not increased or below the threshold value

The method according to the third aspect generally includes providing a sample from the new born subject, from the foetus, or from amniotic fluid encompassing the foetus. According to a particular embodiment of the method according to the third aspect, the amount of proSP-B, or an effective portion thereof, is monitored at certain, e.g. predetermined, time intervals. Samples from the subject may be provided that have been obtained at the corresponding time points.

According to a fourth aspect, the invention provides a method of determining the occurrence of a respiratory disorder in a new born subject or a foetus. The method includes detecting the amount of proSP-B, or an effective portion of proSP-B, in a sample from the new born subject. An increased amount of proSP-B, or the portion thereof, relative to a threshold value, indicates that the new born subject or the foetus is suffering from respiratory disorder. An amount of proSP-B, or of the portion thereof, that is at or below a threshold value, indicates that the new born subject or the foetus, respectively, is not suffering from respiratory disorder.

In some embodiments the method according to the fourth aspect also includes comparing the amount of proSP-B, or the effective portion, in the sample to a threshold value. In some embodiments a method according to the fourth aspect includes determining the occurrence of a respiratory disorder in the new born subject or the foetus if the amount of proSP-B, or the portion thereof, relative to a threshold value is increased, and determining the absence of a respiratory disorder if the amount of proSP-B, or the portion of proSP-B, relative to a threshold value is not increased or below the threshold value.

A method according to the fourth, the fifth and the sixth aspect below generally includes providing a sample from the new born subject. According to a particular embodiment of the fourth, the fifth and the sixth aspect, the respiratory disorder is respiratory distress syndrome.

According to a fifth aspect, the invention provides a method of assessing the risk of occurrence of a respiratory disorder in a new born subject or in a foetus. The method includes determining the amount of proSP-B, or of an effective portion of proSP-B, in a sample from the new born subject. An increased amount of proSP-B, or the portion thereof, relative to a threshold value, indicates an elevated risk of occurrence of a respiratory disorder in the new born subject or the foetus. An amount of proSP-B, or the respective effective portion of proSP-B, that is not above a threshold value or below a threshold value indicates that there is no elevated risk of occurrence of a respiratory disorder in the new born subject or the foetus.

In some embodiments a method according to the fifth aspect includes assessing the risk of occurrence of a respiratory disorder as being elevated if the amount of proSP-B, or the portion thereof, relative to a threshold value is increased, and determining the risk of occurrence of a respiratory disorder as not being elevated if the amount of proSP-B, or the portion thereof, relative to a threshold value is not increased or below the threshold value.

In some embodiments the method according to a fifth aspect includes comparing the amount of proSP-B, or the effective portion, in the sample to the threshold value.

According to a sixth aspect, the invention provides a method of stratifying a new born subject or a foetus for risk of occurrence of a respiratory disorder. The method includes determining the amount of proSP-B, or of an effective portion of proSP-B, in a sample from the new born subject. An increased amount of proSP-B, or the portion thereof, relative to a threshold value, indicates an elevated risk of occurrence of a respiratory disorder in the new born subject or the foetus. An amount ofproSP-B, or the portion thereof, that is not above a threshold value or below a threshold value indicates that there is no elevated risk of occurrence of a respiratory disorder in the new born subject or the foetus.

In some embodiments a method according to the sixth aspect includes stratifying the new born subject or the foetus for risk of occurrence of a respiratory disorder if the amount of proSP-B, or the respective portion of proSP-B, relative to a threshold value is increased, and not stratifying the new born subject or the foetus for risk of occurrence of a respiratory disorder if the amount of proSP-B, or the portion thereof, relative to a threshold value is not increased or below the threshold value.

In some embodiments the method according to the sixth aspect includes comparing the amount of proSP-B, or the effective portion, in the sample to a threshold value.

According to a seventh aspect, the invention relates to the *in-vitro* use of a binding agent specific for proSP-B, or an effective portion thereof, for determining whether a new born subject is in need of respiratory support therapy.

According to a particular embodiment of the use according to the seventh aspect the binding agent is an immunoglobulin or a proteinaceous binding molecule with immunoglobulin-like functions specific for proSP-B.

According to an eighth aspect the invention relates to the *in-vitro* use of a binding agent specific for proSP-B, or an effective portion thereof, for determining whether a foetus will be in need of respiratory support therapy after birth.

According to a particular embodiment of the use according to the eighth aspect the binding agent is an immunoglobulin or a proteinaceous binding molecule with immunoglobulin-like functions. The binding molecule or the immunoglobulin is specific for proSP-B.

According to a ninth aspect the invention relates to the *in-vitro* use of a binding agent specific for proSP-B, or an effective portion thereof, for stratifying a new born subject or a foetus for respiratory support therapy.

According to a particular embodiment of the use according to the ninth aspect the binding agent is an immunoglobulin or a proteinaceous binding molecule with immunoglobulin-like functions specific for proSP-B.

According to a tenth aspect, the invention provides a kit of parts. The kit includes a binding agent, which is specific for proSP-B, or an effective portion of proSP-B. The kit also includes a predetermined amount of proSP-B and/or of the effective portion of proSP-B. The kit may generally be a kit for determining whether a new born subject is in need of respiratory support therapy. The kit may also be a kit for determining whether a foetus will be in need of respiratory support therapy after birth. The kit may also be a kit for stratifying a new born subject or a foetus for respiratory support therapy.

### BRIEF DESCRIPTION OF THE DRAWINGS

The invention will be better understood with reference to the detailed description when considered in conjunction with the non-limiting examples and the accompanying drawings.

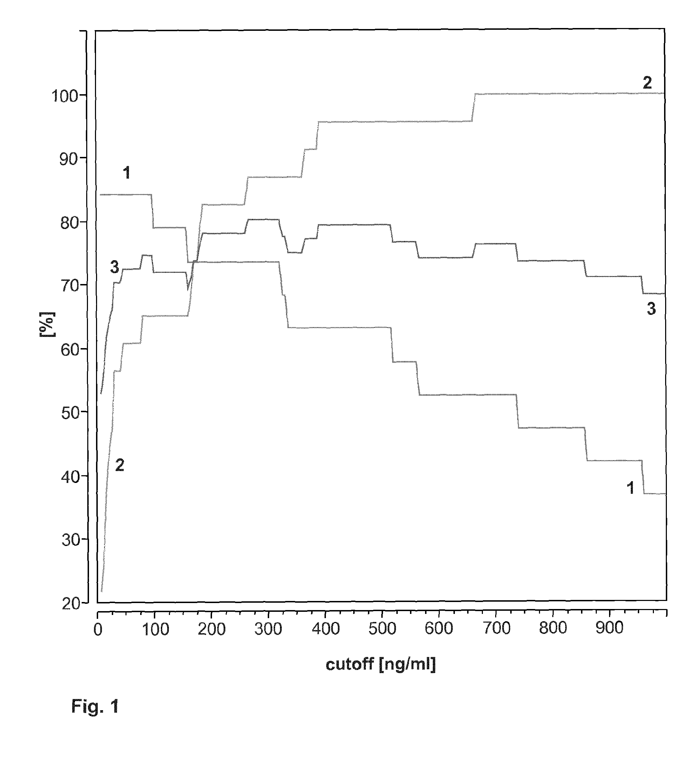

**Figure 1** shows the need for artificial respiration (CPAP or mechanical ventilation) of newborns and pro SP-B [ng/mL] in amniotic fluid, 19 cases and 23 controls, dependence of sensitivity and specificity on cutoff, curve 1 - sensitivity [%], curve 2 - specificity [%], curve 3 - mean of sensitivity, specificity [%]. "Sensitivity" indicates the portion of the confirmed positive test results of the diseased subjects, thereby indicating the probability to have a correct positive test result for a diseased subject. "Specificity" indicates the portion of the confirmed negative test results of the healthy subjects, thereby indicating the probability to have a correct negative result for a healthy subject.

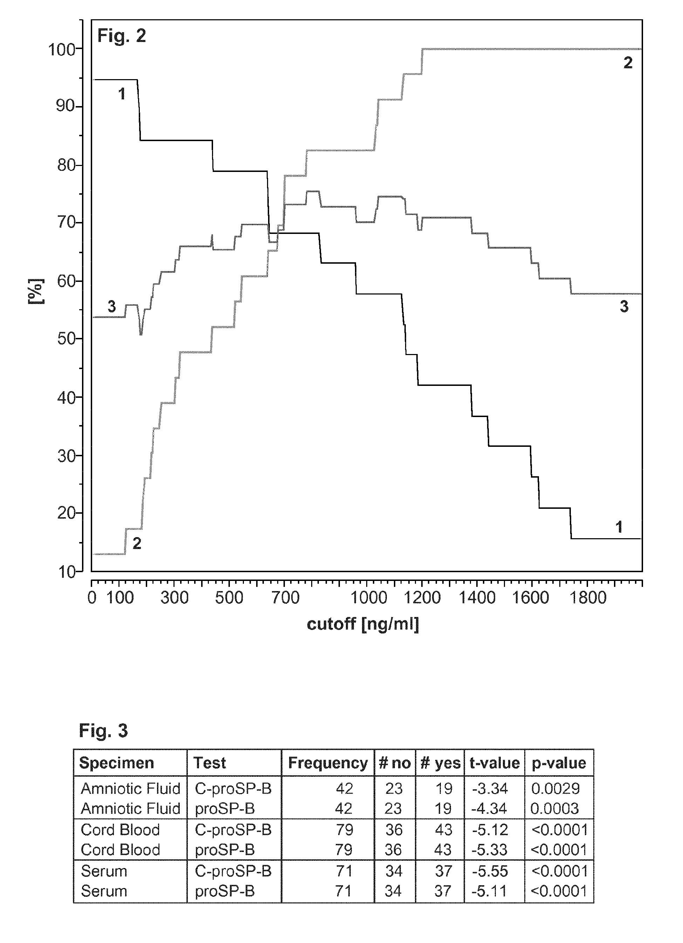

**Figure 2** shows the need for artificial respiration (CPAP or mechanical ventilation) of newborns and the C-terminal fragment of pro SP-B [ng/mL] in amniotic fluid, 19 cases and 23 controls, dependence of sensitivity and specificity on cutoff, curve 1 - sensitivity [%], curve 2 - specificity [%], curve 3 - mean of sensitivity, specificity [%] (cf. above). It is to be noted that full-length pro-SPB was also detected and thus included in the results of this experiment.

**Figure 3** shows an analysis of the statistical significance of the differences in levels of both pro-SPB and of the C-terminal fragment of pro-SPB. The variable used for assessment was the need of artificial respiration, i.e. subjects with mechanical ventilation/CPAP vs. healthy subjects. Compared are data of neonates treated with respiration (CPAP or mechanical ventilation) versus healthy neonates (T-Test with unequal variances). Numbers of "no" and "yes" indicate healthy subjects (no) and subjects in need of CPAP or mechanical ventilation (yes).

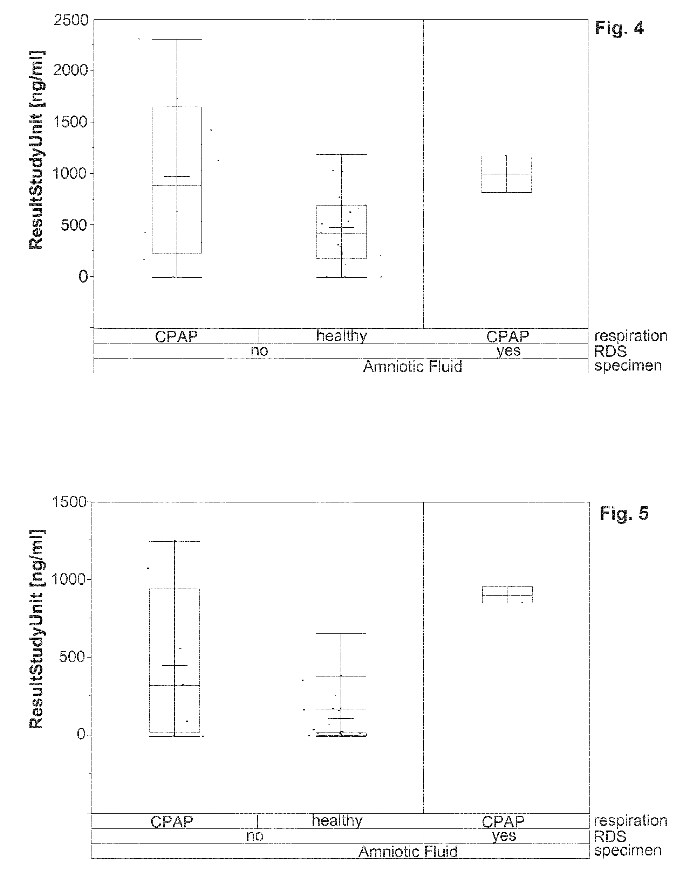

**Figure 4** shows, in the form of box plots, a comparison of levels of the C-terminal fragment of proSP-B (ng/ml) of amniotic fluid of neonates, subdivided into subjects that after birth were under CPAP and healthy subjects [CPAP/healthy] as well as by occurrence of respiratory distress syndrome (RDS) [yes/no].

**Figure 5** depicts, in the form of box plots, a comparison of proSP-B levels (ng/ml) of amniotic fluid of neonates, subdivided into subjects that after birth were under CPAP and healthy subjects [CPAP/healthy] as well as by occurrence of respiratory distress syndrome (RDS) [yes/no].

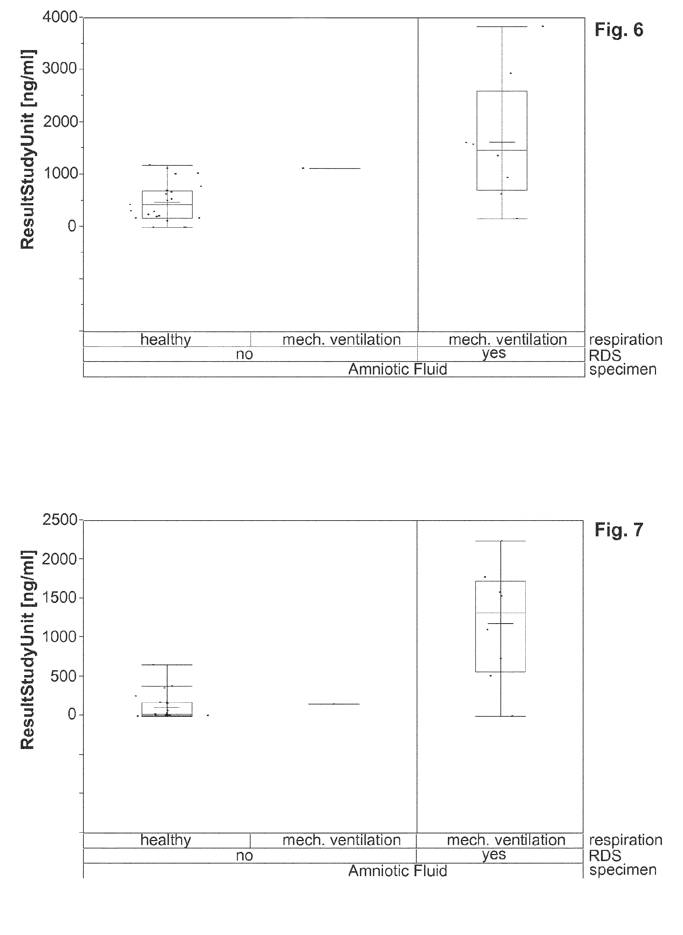

**Figure 6** depicts, in the form of box plots, a comparison of proSP-B levels (ng/ml) of amniotic fluid of neonates, subdivided into subjects that after birth were under mechanical ventilation (treated with mechanical ventilation; mech. ventilation = mechanical ventilation) and healthy subjects [mech.ventilation/healthy] as well as by occurrence of respiratory distress syndrome (RDS) [yes/no].

**Figure 7** depicts, in the form of box plots, a comparison of levels of the C-terminal fragment of proSP-B (ng/ml) of amniotic fluid of neonates, subdivided into subjects that after birth were under artificial respiration (treated with mechanical ventilation; mech. ventilation = mechanical ventilation) and healthy subjects [mech.ventilation/healthy] as well as by occurrence of respiratory distress syndrome (RDS) [yes/no].

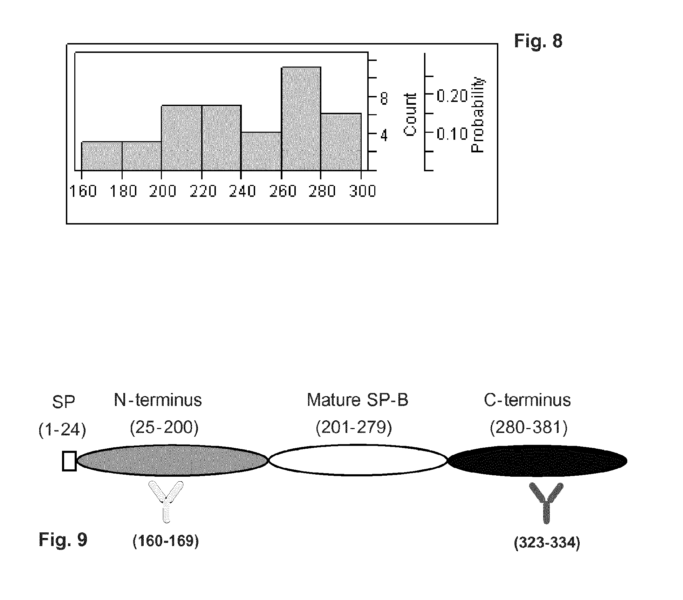

**Figure 8** shows the gestational age, in days, of amniotic fluid used in the Examples of the disclosure, regardless of the status of the subjects after birth.

**Figure 9** depicts the amino acid sequence in correlation to the structure of proSP-B and its C-terminal fragment (SP = signal peptide). Indicated below the schematic representation of the structure are the portions of the sequence that were used to generate antibodies used in the Examples.

### DETAILED DESCRIPTION OF THE INVENTION

The present invention is based on the surprising finding that a newborn or a foetus with an immature lung can be identified on the basis of proSP-B levels. Thereby the need of respiratory support therapy of a newborn or of a foetus after birth can be diagnosed. The invention provides a method for proSP-B or fragments thereof for the early detection of newborns at need for respiratory support therapy. Those skilled in the art will appreciate that the assay may be used on amniotic fluid, e.g. as a point-of care application that allows identifying newborns at need for respiratory support therapy directly at birth or even before birth (puncture for amniotic fluid).

Examples of a respiratory disorder, including respiratory failure, that can be predicted or diagnosed using a method according to the present invention include, but are not limited to, respiratory distress syndrome (RDS), pneumothorax, meconium aspiration syndrome, pneumonia/sepsis, pulmonary interstitial emphysema and bronchopulmonary dysplasia. RDS is the most common lung disorder among infants born prematurely. According to an abstract by Ghafoor et al. (J Coll Physicians Surg Pak. [2003] 13, 5, 271-273), who conducted a study at a hospital of the Pakistan Armed Forces and found 94 neonates out of all live born infants delivered at the hospital to develop RDS. RDS was documented in 1.72% of total live births, and 93.61 % of cases were preterm infants. In a study carried out by Gouyon et al. (Paediatric and Perinatal Epidemiology [2007] 22, 22-30) in Burgundy, France, out of 65,000 term neonates 129 newborns (1.98 ‰) needed artificial respiration for treating respiratory distress resulting from pulmonary disease and 103 infants (1.57 ‰) without severe respiratory disorders needed mechanical ventilation as well. The most common pulmonary illness found in this study was tachypnoea of the newborn, followed by meconium aspiration and respiratory distress syndrome.

As used herein the terms "diagnose" and "diagnosing" refer to determining the occurrence of a respiratory disorder, classifying a respiratory disorder, determining a severity of a respiratory disorder (grade or stage), monitoring the respiratory disorder progression, forecasting an outcome of the respiratory disorder and/or prospects of recovery, monitoring of the outcome of treatment and monitoring the success of respiratory disorder therapy, including respiratory support therapy. "Diagnose" also includes assessing whether a newborn requires respiratory support therapy. It is understood that a respective diagnosis/assessment involves a valuation which may subsequently turn out to be less than 100 % precise for a given individual. Such assessment is in some embodiments to be taken as an indication of the balance of probabilities rather than as a solid predication. The terms "prognosing" and "prognosis" include a prediction of the occurrence and severity of a respiratory disorder, and/or predicting whether a newborn requires and/or will require respiratory support therapy.

Diagnosis of a respiratory disorder such as RDS may be confirmed by other means such as chest x-ray (CXR). A CXR can also be carried out to check the position of an endotracheal (ET) tube before commencing e.g. administration of surfactant (cf. below). A further suitable technique is blood gas analysis. The need for respiratory support therapy may additionally also be assessed on the basis of symptoms such as cyanosis (bluish skin), grunting, retraction and/or tachypnea.

The term "respiratory support therapy" as used herein refers to both ventilatory and non-ventilatory forms of support therapy. Ventilatory forms of support therapy include any form of artificial respiration, including artificial assistance or artificial replacement for respiration, which may be based on mechanical or non-mechanical means in order to assist, maintain or create a flow of gas into and out of the lungs of a subject. As used herein, the term "mechanical ventilation" is used for invasive ventilation, in line with the "European Consensus Guidelines on the Management of Neonatal Respiratory Distress Syndrome in Preterm Infants - 2010 Update" (Sweet, D.G., et al., Neonatology (2010) 97, 402-417). In contrast to mechanical ventilation, non-invasive ventilation is understood as providing ventilatory support via the patient's upper airway using a device such as a face mask, a nasal mask, a helmet and/or a ventilator such as a negative pressure ventilator. Non-invasive ventilation generally refers to providing ventilatory support through a subject's upper airway. Invasive ventilation generally bypasses the upper airway by means of e.g. a tracheal tube, a laryngeal mask, or tracheostomy.

Non-invasive ventilation thus includes continuous positive airway pressure (CPAP), bilevel positive airway pressure (BIPAP), proportional assist ventilation (PAV), non-invasive positive pressure ventilation (NPPV), and external negative pressure. Non-invasive ventilation may also be carried out as volume limited ventilation or using rocking beds. Mechanical ventilation is a supportive intervention that may be life saving in critically ill subjects, however, it involves a significant risk of morbidity and mortality. The use of non-invasive ventilation may decrease the risk of various serious problems such as ventilator-associated pneumonia, sedation-related problems, and sinusitis.

Established methods of non-invasive ventilation include, but are not limited to, volume controlled continuous mandatory ventilation, including controlled mechanical ventilation (CMV) or volume controlled continuous mandatory ventilation, volume controlled intermittent mandatory ventilation (VC-IMV), pressure controlled continuous mandatory ventilation (PC-CMV), pressure controlled intermittent mandatory ventilation (also called synchronized intermittent mechanical ventilation, SIMV), high frequency ventilation and continuous spontaneous ventilation, including pressure support ventilation (PSV) or continuous positive airway pressure (CPAP). As indicated above, the term "artificial respiration" refers to any form of ventilatory support, and includes both mechanical ventilation and non-invasive ventilation such as CPAP. Mechanical ventilation, i.e. invasive ventilation, may for instance be achieved via tracheal intubation. Non-ventilatory forms of support therapy may include providing a gas for breathing that differs in composition from the composition of air, for example by having an increased amount of oxygen when compared to air. It will be understood that mechanical ventilation and non-invasive ventilation may be combined with non-ventilatory support therapy.

Once a subject has been identified as being in need of respiratory support therapy by a method according to the present invention a decision can be taken as to start such therapy. One of the hallmarks of RDS is the absence or reduction of surfactant. Respiratory support therapy may in this regard include administration of lung surfactant and/or surfactant substitutes (e.g., chemically modified forms of naturally existing surfactants), typically on an intubated newborn. In some embodiments it may be sufficient to use nebulized liquid surfactant without intubation. As mentioned above, respiratory support therapy may further include non-invasive ventilation, e.g. continuous positive airway pressure, nasal continuous positive airway pressure (nCPAP) or nasal intermittent positive pressure ventilation (NIPPV), for instance via ventilator and/or by means of self-inflating or flow-inflating bags, administration of low flow oxygen and/or ventilator support such as mechanical ventilation via a face mask or through tracheal intubation. Modes of ventilation may be selected as required. Typically an apparatus for mechanical ventilation allows the selection of at least three basic modes, which are spontaneous, assisted or controlled. Spontaneous ventilation without other modes of ventilation, refers to a subject breathing at his/her own pace, but other interventions may affect other parameters of ventilation including the tidal volume and the baseline pressure, above ambient, within the system. In assisted ventilation, the patient initiates the inhalation by lowering the baseline pressure by varying degrees, and then the ventilator "assists" the patient by completing the breath by the application of positive pressure. During controlled ventilation, the subject is unable to breathe spontaneously or initiate a breath, and is therefore dependent on the ventilator for every breath.

Such a measure may be accompanied by arterial blood gas assessment. Respiratory support therapy may also include any of correction of hypothermia, hypoglycaemia, acidosis and hypovolaemia, as required. Where a foetus is diagnosed to be in need of respiratory support therapy after birth various measures may likewise be considered by the practitioner. For instance a hospital may be selected for birth where appropriate equipment and knowledge on RDS after birth is available. In case of expected preterm delivery a delay of birth may in some embodiments be possible. In such cases prenatal corticosteroid and/or antibiotic administration may also be considered.

As indicated above, surfactant contains about 85 to 90 % lipids and about 10 to 15 % protein. The four specific surfactant proteins known (supra), SP-A, SP-B, SP-C and SP-D, provide about 2 to 5 % of the weight of surfactant. Pulmonary surfactant is synthesized in epithelial cells of the alveoli and stored in the lamellar body. It is secreted via exocytosis of the lamellar body.

As explained below, proSP-B is an immature form of mature SP-B, with the latter being secreted into the lung. It is so far not known what regulatory mechanisms control the conversion of proSP-B to mature SP-B, neither is it clear why proSP-B appears to be secreted into the lung of foetuses and newborns, but has reportedly not been found in adults (Hamvas, A. et al., Neonatalogy (2009) 95, 2, 117-124), and why it can be found in their blood. Hamvas et. al. (ibid.) have suggested that components of the SP-B maturation pathway such as proteases may not be fully functional in foetuses and newborns.

Any function of C-terminal fragments of proSP-B has so far been unclear and their potential as a possible marker for alveolar and lung maturity has so far been not been considered. Neither has a ratio of C-terminal fragments of proSP-B to proSP-B and/or SP-B for respiratory diseases been analysed. Pryhuber et al. (Pryhuber, G.S., et al., Mol Gen Metabol (1998) 64, 217-228) describe the role of Surfactant protein B and its proproteins as follows: " In humans, the SP-B mRNA and proprotein are detected as early as 14-15 weeks of gestation prior to detection of mature surfactant ... However, mature SP-B protein is undetectable in lung tissue prior to 19 weeks of gestation. In addition, mature SP-B protein is not seen in human amniotic fluid until 31 to 33 weeks of gestation (...). The early appearance of SP-B proprotein in fetal lung suggests that the proprotein may have a function in early development which is not directly related to surface tension reduction."

One method of the invention is a method of assessing whether a newborn is in need of respiratory support therapy or whether a foetus will be in need of respiratory support therapy after birth. A related method is a method of screening one or more newborn individuals or foetuses for susceptibility to a respiratory disorder such as RDS. A further related method is a method of monitoring the susceptibility of a respiratory disorder related condition in a subject. In this regard the term "susceptibility" as used herein refers to the degree of risk of a subject to an indicated condition, in the context of the present invention generally a pathological condition, including a disease or disorder. The term "susceptibility to RDS" refers to the likelihood that RDS will occur in a subject. As noted above, it is understood that a respective diagnosis/assessment involves a valuation which may subsequently turn out to be less than 100 % precise for a given individual. Such assessment is in some embodiments to be taken as an indication of the balance of probabilities rather than as a solid predication.

A respective method according to the present invention involves analysis of a sample from the subject *in vitro.* Typically the sample is or includes body fluid from the subject. Where the subject is a foetus the sample may also include or consist of body fluid encompassing the foetus. The sample may in some embodiments be or include amniotic fluid, or it may be or include cord blood. The sample may in some embodiments be or include whole blood, plasma, lymph, serum or tracheal aspirate. In some embodiments the sample may be or include a whole blood sample, a plasma sample, a lymph sample, a serum sample and a tracheal aspirate sample. In some embodiments the method may include providing a sample from the subject. In some embodiments the method may include providing a sample from body fluid encompassing the subject before birth or from the foetus. Where the sample is or includes amniotic fluid, the sample has generally been taken at gestagation week 23 or later, such as at gestagation week 26, gestagation week 31, gestagation week 36, gestagation week 41 or later.

The sample may have been taken at any desired point in time before carrying out the method of the invention. Generally the time interval between taking the sample and carrying out the method of the invention is selected to allow analysis of intact proSP-B or an effective portion of proSPB. In some embodiments the sample has been taken on the same day or within the last two days such as the day before. The sample may for instance have been obtained 72 hours or less, such as 60 hours or less, 48 hours or less, 32 hours, 24 hours, 12 hours before the method of the invention is being carried out and been kept at about room temperature, i.e. about 18 °C. The sample may also have been kept at a temperature below room temperature, for instance be kept at a temperature in the range from about 2 °C to about 18 °C, such as from about 4 °C to about 18 °C, including at about 15 °C or below, or at or below 10 °C, such as about 4 °C or about 8 °C. In some embodiments the sample has been taken within the same week, such as 7 days or less, 6 days or less, 5 days or less, 4 days or less, 3 days or less, 2 days or less, 32 hours or less, 24 hours or less, 12 hours or less before the method of the invention is being carried out and been kept in fluid form at a temperature of about 12 °C or less, such as about 10 °C or less, about 8 °C or less, including e.g. about 4°C. The subject from which/whom the sample has been obtained is generally a mammal such as a rabbit, a mouse, a rat, a Guinea pig, a hamster, a dog, a pig, a cow, a goat, a sheep, a horse, a macaque, a gibbon or a human.

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and below of up to 10%, such as up to 5%, up to 2%, up to 1%, up to 0.5 % or up to 0.1 % of a given value.

In some embodiments the sample from the individual is a frozen sample. Generally the sample is frozen within the above detailed time intervals, e.g. 0 to about 48 or 0 to about 42 hours, and/or at the above exemplified time points, such as about 48 hours, about 36 hours or less, after the sample has been obtained from the individual and kept at room temperature. Where the sample is at least essentially void of cells such as in case of a serum or a plasma sample, the sample may be brought to a temperature below freezing point. Where the sample includes cells that are not intended to be depleted from the sample it may in some embodiments be desired to maintain these cells at least essentially intact for carrying out a method according to the invention. In such an embodiment a frozen sample may be formed by freezing an obtained sample after adding a cryoprotective agent such as DMSO, glycerol and/or hydroxyethyl starch. As an illustrative example DMSO may be used in a final concentration in the range from about 2% to about 10 %, such as about 2%, about 4%, about 5% or about 10% DMSO. Typically the sample is then frozen at a controlled rate to a temperature less than -50°C, whereafter the sample may for instance be stored, including long-term storage, at a temperature below -130°C such as -160°C, e.g. in liquid nitrogen for extended periods of time.

As indicated above, in some embodiments the sample may be depleted of cells. Depletion of cells from the sample may in some embodiments include general cell removal techniques such as centrifugation, filtration or cell chromatography. In some embodiments of a method according to the invention a sample as provided from the individual is depleted of erythrocytes, in some embodiments at least essentially cleared of erythrocytes, if required. Depletion or removal of erythrocytes may for example in some embodiments be required in case the sample is a whole blood sample or a blood cell sample. Lysis of erythrocytes may be carried out osmotically or chemically. Osmotic lysis is suitable in the contest of the present invention since erythrocytes lyse at an osmolarity at which leukocytes remain intact. In the art typically a solution of ammonium chloride is used for osmotic lysis, which may further include potassium bicarbonate and/or EDTA. A commercially available reagent may be used, such as the FCM Lysing solution by Santa Cruz (order no sc-3621), Erythrolyse Red Blood Cell Lysing Buffer by AbD Serotec or RBC Lysis Solution by 5 PRIME. Chemical lysis of erythrocytes may for example be achieved using an organic solvent such as diethylether or chloroform, and/or a surfactant, a copper containing solution or via adding one of certain bacterial or animal toxins. After lysis of erythrocytes the remaining blood cells may be collected, for example by means of centrifugation.

A method according to the invention includes detecting the amount of pro-Pulmonary surfactant-associated protein B (proSP-B), or an effective portion thereof, in the sample. The protein proSP-B may be any respective variant or isoform of the respective species, e.g. human. The protein may for example be the human protein of the Swissprot/Uniprot accession number P07988 (version 152 as of 11 July 2012, SEQ ID NO: 1). This protein may for instance be encoded by the mRNA of GenBank accession number NM₋198843 (version NM_198843.2; GI:288856296) or the mRNA of GenBank accession number NM_000542 (version NM_000542.3; GI: 288856298). The corresponding gene may in some embodiments be the human gene SFTPB with the GenBank Gene ID: 6439 (as of 04 February 2012). The protein may in some embodiments be the rabbit protein of the Swissprot/Uniprot accession number P15285 (version 96 as of 19 October 2011). The protein may also be the rat protein of the Swissprot/Uniprot accession number P22355 (version 93 as of 21 September 2011). The protein may in some embodiments be encoded by the rat gene SFTPB with the GenBank Gene ID: 192155 (as of 04 February 2012). In some embodiments the protein may be the bovine protein of the Swissprot/Uniprot accession number P15781 (version 88 as of 21 September 2011). The protein may in some embodiments be encoded by the bovine gene SFTPB with the GenBank Gene ID: 507398 (as of 28 February 2012). The protein may also be the mouse protein of the Swissprot/Uniprot accession number P50405 (version 89 as of 16 November 2011). In some embodiments the protein may be the dog protein of the Swissprot/Uniprot accession number P17129 (version 90 as of 21 September 2011). The protein may in some embodiments be the sheep protein of the Swissprot/Uniprot accession number Q9TU81 (version 40 as of 5 October 2011). The protein may also be the porcine protein of the Swissprot/Uniprot accession number P15782 (version 73 as of 27 July 2011). In some embodiments the protein may be the Guinea pig protein of the Swissprot/Uniprot accession number 035489 (version 53 as of 21 September 2011). Several natural variants of the human proSP-B protein of Swissprot/Uniprot accession number P07988 are known to exist, due to single nucleotide polymorphism. Such variants are listed under Swissprot/Uniprot entry P07988 as e.g. VAR_006948, VAR 013099, VAR_006950, VAR_006949, VAR_036856 and VAR_013100. The term proSP-B as used herein is meant to include such variants.

The term "variant" as used herein refers to a peptide or protein with an amino acid sequence which differs from the protein at issue due to one or more amino acid substitution(s), deletion(s) and/or addition(s). A variant may have an amino acid sequence with at least about 60 %, at least about 70 %, at least about 75 %, at least about 80 %, at least about 85 %, at least about 90 %, at least about 92 %, at least about 95 %, at least about 96 %, at least about 97 %, at least about 98 % or at least about 99 % sequence identity with a corresponding naturally occurring form of the respective protein. A variant may be an allelic variant or any other species specific homolog, paralog, or ortholog. A variant may also be a fragments of the corresponding protein, e.g. proSP-B, including a fragment of the aforementioned types of variants as long as these fragments have the essential immunological and biological properties as the known predominant form of the corresponding naturally occurring protein. A variant may for instance have immunological properties (i.e. epitope composition) comparable to those of a known protein form. Thus, a variant can typically be recognized by a binding partner used for determination of the concentration of the protein. A fragment is in some embodiments a degradation product of the known form of the protein, e.g. proSP-B. A variant may also differ due to posttranslational modifications such as phosphorylation or myristylation or have a different glycosylation pattern.

An "epitope" is antigenic and thus an epitope may also be taken to define an "antigenic structure" or "antigenic determinant". Thus, a binding domain of an immunoglobulin or of a proteinaceous binding molecule with immunoglobulin-like functions is an "antigen-interaction-site". The term "antigen-interaction-site" defines, in accordance with the present invention, a motif of a polypeptide, which is able to specifically interact with a specific antigen or a specific group of antigens, e.g. proSP-B in different species. Said binding/interaction is also understood to define a "specific recognition".

The term "epitope" also refers to a site on an antigen such as proSP-B with which an immunoglobulin or a proteinaceous binding molecule with immunoglobulin-like functions forms a complex. In some embodiments, an epitope is a site on a molecule against which an immunoglobulin or a proteinaceous binding molecule with immunoglobulin-like functions will be produced and/or to which an antibody will bind. For example, an epitope can be recognized by an immunoglobulin or a proteinaceous binding molecule with immunoglobulin-like functions. The epitope may be a "linear epitope", which is an epitope where an amino acid primary sequence contains the epitope recognized. A linear epitope typically includes at least 3, and more usually, at least 5, for example, about 8 to about 10 amino acids in a unique sequence. The epitope may also be a "conformational epitope", which in contrast to a linear epitope, is an epitope where the primary sequence of the amino acids that includes the epitope is not the sole defining component of the epitope recognized (e.g., an epitope wherein the primary sequence of amino acids is not necessarily recognized by the antibody defining the epitope). Typically a conformational epitope includes a larger number of amino acids than a linear epitope. With regard to recognition of conformational epitopes, an immunoglobulin or a proteinaceous binding molecule with immunoglobulin-like functions recognizes a 3-dimensional structure of the antigen, such as a peptide or protein or a fragment thereof. As an illustrative example, when a protein molecule folds to form a three dimensional structure, certain amino acids and/or all or portions of the polypeptide backbone forming the conformational epitope become juxtaposed, allowing an antibody to recognize the epitope. Methods of determining conformation of epitopes include, but are not limited to, x-ray crystallography, 2-dimensional nuclear magnetic resonance spectroscopy, site-directed spin labeling and electron paramagnetic resonance spectroscopy.

"Percent (%) sequence identity" with respect amino acid sequences disclosed herein is defined as the percentage of amino acid residues in a candidate sequence that are identical with the amino acid residues in a reference sequence, e.g. a proSP-B molecule, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The same applies to nucleotide sequences disclosed herein.

Mature SP-B is secreted into the alveolar space where it is mainly found as a homodimer. SP-B is a protein that is included in lung surfactant, a complex mixture of proteins and lipids. SP-B is essential for lung surfactant function, including the regulation of surface tension in the lungs during breathing. Lung surfactant forms a continuous film at the liquid-air interface of the alveoli, which is fundamental to breathing. It allows normal breathing by reducing the surface tension at the air/water interface in alveoli. It further provides the first line of defense against inhaled microbes in the lungs. SP-B enhances the rate of phospholipid adsorption to the air/water interface, decreases surface tension by interfering with the attractive forces acting between water molecules and has anti-inflammatory properties. SP-B is thought to promote the rapid formation of the alveolar film. Sp-B is amphiphatic and to a large extent hydrophobic. Just as the hydrophobic Sp-C it is directly interspersed into the lipid component of the lung surfactant.

Hydrophobic ("water-fearing") matter, has a tendency to separate from water. In contrast thereto, hydrophilic ("water-loving") matter generally contains molecules or moieties which can form dipole-dipole interactions with water molecules and thus have a high wettability for water (see also below). Hydrophilic compounds generally readily dissolve in water. Usually hydrophobic matter is apolar and possesses an even distribution of electron density. A related term is the indication lipophilic ("fat-loving"). Lipophilic matter attracts non-polar organic compounds, such as oils, fats, or greases. It is understood that on a general basis the terms "hydrophobic" and "lipophilic" are not necessarily synonymous. For example perfluorocarbon compounds are both hydrophobic and oleophobic, i.e. lack an affinity for oils. Such compounds accordingly have a tendency to separate from both water and hydrocarbons (though the latter to a lesser extent than from water). However, in the context of the present invention, "hydrophobic" and "lipophilic" can generally be used interchangeably. An amphiphilic, also called amphiphiphatic, compound has both hydrophilic and hydrophobic properties. Generally such a compound has hydrophilic portions and hydrophobic portions. In the case of Sp-B, the mature protein has an amphiphatic helix close to its N-terminus.

The mature form of surfactant-associated protein B differs from proSP-B in the lack of an N-terminal signal peptide, the lack of a subsequent N-terminal propeptide and the lack of a C-terminal propeptide. Further, proSP-B is being glycosylated, whereas the corresponding glycosylation sites are not present in mature SP-B. Mature human SP-B has a length of 70 amino acids, while human proSP-B has an additional 176 N-terminal amino acids and additional 102 C-terminal amino acids.

An effective portion of proSP-B is a peptide that has, including consists of, a sequence of amino acids that defines the C-terminal portion of proSP-B, such as the C-terminal fourth of proSP-B. It is recalled that the C-terminus of proSP-B differs from the C-terminus of mature SP-B in that proSP-B has a propeptide sequence, which defines amino acids 280-381 of the human protein of the Swissprot/Uniprot accession number P07988 (SEQ ID NO: 2). In typical embodiments an effective portion of proSP-B has a portion of at least about 25, including at least about 30, at least about 35, at least about 40, at least about 45 or at least about 50 amino acids of the Saposin B-type 3 domain, which is defined by amino acids 295-370 of Swissprot/Uniprot accession number P07988 (SEQ ID NO: 3). Typically a respective sequence of amino acids is located within the N-terminal half of the Saposin B-type 3 domain. Typically an effective portion of proSP-B further includes 5 or more, including 10 or more amino acids of the C-terminal end of the Saposin B-type 2 domain of proSP-B, which is defined by the amino acid positions corresponding to amino acids 204-281 of the sequence of Swissprot/Uniprot accession number P07988 (SEQ ID NO: 4). In one embodiment an effective portion of proSP-B includes, including consists of, the amino acid positions that correspond to amino acid positions 279 to 381 of the sequence of Swissprot/ Uniprot accession number P07988 (SEQ ID NO: 5). In one embodiment an effective portion of proSP-B includes, including consists of, the amino acid positions that correspond to amino acid positions 285 to 334 of the sequence of Swissprot/Uniprot accession number P07988 (SEQ ID NO: 9). In some embodiments the effective portion of proSP-B is monomeric. In some embodiments the effective portion of proSP-B is dimeric, for example defining a homodimer.

In a method according to the invention the amount of proSP-B, or an effective portion thereof, is detected in the sample. The word "detect" or "detecting" when used herein in combination with the word "amount" is understood to generally refer to a quantitative rather than on a qualitative level. In this regard the words "value," "amount" and "level" are used interchangeably herein. Accordingly, the method includes a quantification of proSP-B, i.e. the level of proSP-B, or an effective portion thereof, is determined. Such a measurement may for instance rely on spectroscopic (e.g., NMR), surface plasmon resonance, photochemical, photometric, fluorometric, radiological, enzymatic or thermodynamic means. The measurement used is generally selected to be of a sensitivity that allows detection of proSP-B, or an effective portion thereof, at levels in the range of the threshold value, in particular of a sensitivity that allows determining whether proSP-B levels (or levels of an effective portion thereof) are above or below the threshold value. Typically a binding partner of proSP-B, including of an effective portion thereof, may be contacted with the sample. In some embodiments a complex is allowed to form between proSP-B, or the effective portion of proSP-B, and the binding partner. The amount of the binding partner binding to, e.g. forming a complex with, proSP-B, or an effective portion thereof, may be quantified in order to assess the amount of proSP-B/ effective portion of proSP-B, present. Before detecting the amount of binding partner, non-bound binding partner may in some embodiments be removed from the sample. A respective binding partner of proSP-B or an effective portion thereof, herein also referred to as a "binding agent" for proSP-B or for an effective portion thereof, may be used in combination with a detectable marker. In some embodiments a detectable marker may be encompassed in the binding agent/binding partner of proSP-B or an effective portion thereof.

Such a binding partner of proSP-B, or an effective portion thereof, has a detectable affinity and specificity for proSP-B, or the effective portion thereof. Typically, binding is considered specific when the binding affinity is higher than 10⁻⁶ M. A binding partner of proSP-B, or an effective portion thereof, has in some embodiments an affinity of about 10⁻⁸ M or higher or of about 10⁻⁹ M or higher. The term "specific" is understood to indicate that the binding partner is directed against, binds to, or reacts with proSP-B, or an effective portion thereof. Thus, being directed to, binding to or reacting with includes that the binding partner specifically binds to proSP-B, or the effective portion thereof. The term "specifically" in this context means that the binding partner reacts with proSP-B or/and a portion thereof, but at least essentially not with another protein. The term "another protein" includes any protein, including proteins closely related to or being homologous to proSP-B against which the binding partner is directed to. The term "does not essentially bind" means that the binding partner does not bind another protein, i.e., shows a cross-reactivity of less than about 30%, such as less than about 20%, less than about 10%, including less than about 9, 8, 7, 6 or 5%, when compared to the affinity to proSP-B or an effective portion thereof. Whether the binding partner specifically reacts as defined herein above can easily be tested, inter alia, by comparing the reaction of a respective binding partner with proSP-B (or an effective portion of proSP-B) and the reaction of the binding partner with (an) other protein(s). "Specific binding" can also be determined, for example, in accordance with Western blot analysis, ELISA-, RIA-, ECL-, IRMA-tests, FACS, IHC and peptide scans.

A respective binding partner of proSP-B, or an effective portion thereof, may be an immunoglobulin, a fragment thereof, a proteinaceous binding molecule with immunoglobulin-like functions or an aptamer. Examples of (recombinant) immunoglobulin fragments are Fab fragments, Fv fragments, single-chain Fv fragments (scFv), diabodies or domain antibodies (Holt, L.J., et al., Trends Biotechnol. (2003), 21, 11, 484-490). An example of a proteinaceous binding molecule with immunoglobulin-like functions is a mutein based on a polypeptide of the lipocalin family (WO 03/029462, Beste et al., Proc. Natl. Acad. Sci. USA (1999) 96, 1898-1903). Lipocalins, such as the bilin binding protein, the human neutrophil gelatinase-associated lipocalin, human Apolipoprotein D or glycodelin, possess natural ligand-binding sites that can be modified so that they bind to selected small protein regions known as haptens. Examples of other proteinaceous binding molecules are the so-called glubodies (see WO 96/23879), proteins based on the ankyrin scaffold (Mosavi, L.K., et al., Protein Science (2004) 13, 6, 1435-1448) or crystalline scaffold (WO 01/04144) the proteins described in Skerra, J Mol. Recognit. (2000) 13, 167-187, a protein based on a plurality of low-density lipoprotein receptor class A (LDLR-A) domains, an AdNectin, a tetranectin and an avimer. Avimers contain so called A-domains that occur as strings of multiple domains in several cell surface receptors (Silverman, J, et al., Nature Biotechnology (2005) 23, 1556-1561). A molecule that forms a complex with a binding partner of proSP-B may likewise be an immunoglobulin, a fragment thereof or a proteinaceous binding molecule with immunoglobulin-like functions, as explained above. Thus, in an exemplary embodiment detecting the amount of proSP-B may be carried out using a first antibody or antibody fragment capable of specifically binding proSP-B, as well as a second antibody or antibody fragment capable of specifically binding the first antibody or antibody fragment.

The binding partner of proSP-B, or an effective portion thereof, may be immobilised on a surface (vide infra) such as the surface of a particle such as a metal containing bead. The binding partner may be immobilised by any means. It may be immobilised on a portion or the entire area of a surface. An illustrative example is the mechanical spotting of a nucleic acid capture molecule onto a metal surface. This spotting may be carried out manually, e.g. by means of a pipette, or automatically, e.g. by means of a micro robot. As an illustrative example, a protein capture molecule, a peptide capture molecule or the polypeptide backbone of a PNA capture molecule may be covalently linked to a gold surface via a thio-ether-bond.

The term "antibody" as used herein, is understood to include an immunoglobulin and an immunoglobulin fragment that is capable of specifically binding a selected protein, e.g. proSP-B, as well as a respective proteinaceous binding molecule with immunoglobulin-like functions. As an illustrative example an antibody may be a camel heavy chain immunoglobulin. As a few further non-limiting examples, an antibody may be an EGF-like domain, a Kringle-domain, a fibronectin type I domain, a fibronectin type II domain, a fibronectin type III domain, a PAN domain, a G1a domain, a SRCR domain, a Kunitz/Bovine pancreatic trypsin Inhibitor domain, tendamistat, a Kazal-type serine protease inhibitor domain, a Trefoil (P-type) domain, a von Willebrand factor type C domain, an Anaphylatoxin-like domain, a CUB domain, a thyroglobulin type I repeat, an LDL-receptor class A domain, a Sushi domain, a Link domain, a Thrombospondin type I domain, an immunoglobulin domain or a an immunoglobulin-like domain (see above for further examples). In some embodiments an antibody is an aptamer, including a Spiegelmer®, described in e.g. WO 01/92655. An aptamer is typically a nucleic acid molecule that can be selected from a random nucleic acid pool based on its ability to bind a selected other molecule such as a peptide, a protein, a nucleic acid molecule a or a cell. Aptamers, including Spiegelmers, are able to bind molecules such as peptides, proteins and low molecular weight compounds. Spiegelmers® are composed of L-isomers of natural oligonucleotides. Aptamers are engineered through repeated rounds of *in vitro* selection or through the SELEX (systematic evolution of ligands by exponential enrichment) technology. The affinity of Spiegelmers to their target molecules often lies in the pico- to nanomolar range and is thus comparable to immunoglobulins. An aptamer may also be a peptide. A peptide aptamer consists of a short variable peptide domain, attached at both ends to a protein scaffold.

By "fragment" in reference to a polypeptide such as an immunoglobulin or a proteinaceous binding molecule is meant any amino acid sequence present in a corresponding polypeptide, as long as it is shorter than the full length sequence and as long as it is capable of performing the function of interest of the protein - in the case of an immunoglobulin specifically binding to the desired target, e.g. antigen (proSP-B, for example). The term "immunoglobulin fragment" refers to a portion of an immunoglobulin, often the hypervariable region and portions of the surrounding heavy and light chains that displays specific binding affinity for a particular molecule. A hypervariable region is a portion of an immunoglobulin that physically binds to the polypeptide target.

An immunoglobulin may be monoclonal or polyclonal. The term "polyclonal" refers to immunoglobulins that are heterogenous populations of immunoglobulin molecules derived from the sera of animals immunized with an antigen or an antigenic functional derivative thereof. For the production of polyclonal immunoglobulins, one or more of various host animals may be immunized by injection with the antigen. Various adjuvants may be used to increase the immunological response, depending on the host species. "Monoclonal immunoglobulins" or "Monoclonal antibodies" are substantially homogenous populations of immunoglobulins to a particular antigen. They may be obtained by any technique which provides for the production of immunoglobulin molecules by continuous cell lines in culture. Monoclonal immunoglobulins may be obtained by methods well known to those skilled in the art (see for example, Köhler et al., Nature (1975) 256,495-497, and U.S. Patent No. 4,376,110). An immunoglobulin or immunoglobulin fragment with specific binding affinity only for proSP-B, or an effective portion thereof, can be isolated, enriched, or purified from a prokaryotic or eukaryotic organism. Routine methods known to those skilled in the art enable production of both immunoglobulins or immunoglobulin fragments and proteinaceous binding molecules with immunoglobulin-like functions, in both prokaryotic and eukaryotic organisms.

In more detail, an immunoglobulin may be isolated by comparing its binding affinity to a protein of interest, e.g. proSP-B, with its binding affinity to other polypeptides. Humanized forms of the antibodies of the present invention may be generated using one of the procedures known in the art such as chimerization or CDR grafting. In general, techniques for preparing monoclonal antibodies and hybridomas are well known in the art. Any animal such as a goat, a mouse or a rabbit that is known to produce antibodies can be immunized with the selected polypeptide, e.g. proSP-B. Methods for immunization are well known in the art. Such methods include subcutaneous or intraperitoneal injection of the polypeptide. One skilled in the art will recognize that the amount of polypeptide used for immunization and the immunization regimen will vary based on the animal which is immunized, including the species of mammal immunized, its immune status and the body weight of the mammal, as well as the antigenicity of the polypeptide and the site of injection.

The polypeptide may be modified or administered in an adjuvant in order to increase the peptide antigenicity. Methods of increasing the antigenicity of a polypeptide are well known in the art. Such procedures include coupling the antigen with a heterologous protein (such as globulin or β-galactosidase) or through the inclusion of an adjuvant during immunization.

Typically, the immunized mammals are bled and the serum from each blood sample is assayed for particular antibodies using appropriate screening assays. As an illustrative example, anti-proSP-B immunoglobulins may be identified by immunoprecipitation of ¹²⁵I-labeled cell lysates from proSP-B expressing cells (see, Sanchez-Madrid et al., 1986 and Hemler et al., 1987). Anti-proSP-B immunoglobulins may also be identified by flow cytometry, e.g., by measuring fluorescent staining of Ramos cells incubated with an antibody believed to recognize proSP-B.

For monoclonal immunoglobulins, lymphocytes, typically splenocytes, from the immunized animals are removed, fused with an immortal cell line, typically myeloma cells, such as SP2/0-Ag14 myeloma cells, and allowed to become monoclonal immunoglobulin producing hybridoma cells. Typically, the immortal cell line such as a myeloma cell line is derived from the same mammalian species as the lymphocytes. Illustrative immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using 1500 molecular weight polyethylene glycol ("PEG 1500"). Hybridoma cells resulting from the fusion may then be selected using HAT medium, which kills unfused and unproductively fused myeloma cells (unfused splenocytes die after several days because they are not transformed).

Any one of a number of methods well known in the art can be used to identify a hybridoma cell which produces an immunoglobulin with the desired characteristics. Typically the culture supernatants of the hybridoma cells are screened for immunoglobulins against the antigen. Suitable methods include, but are not limited to, screening the hybridomas with an ELISA assay, Western blot analysis, or radioimmunoassay (Lutz et al., Exp. Cell Res. [1988] 175, 109-124). Hybridomas prepared to produce anti-proSP-B immunoglobulins may for instance be screened by testing the hybridoma culture supernatant for secreted antibodies having the ability to bind to a recombinant proSP-B expressing cell line. To produce antibody homologs which are within the scope of the invention, including for example, anti-proSP-B antibody homologs, that are intact immunoglobulins, hybridoma cells that tested positive in such screening assays can be cultured in a nutrient medium under conditions and for a time sufficient to allow the hybridoma cells to secrete the monoclonal immunoglobulins into the culture medium. Tissue culture techniques and culture media suitable for hybridoma cells are well known in the art. The conditioned hybridoma culture supernatant may be collected and for instance the anti-proSP-B immunoglobulins optionally be further purified by well-known methods. Alternatively, the desired immunoglobulins may be produced by injecting the hybridoma cells into the peritoneal cavity of an unimmunized mouse. The hybridoma cells proliferate in the peritoneal cavity, secreting the immunoglobulin which accumulates as ascites fluid. The immunoglobulin may be harvested by withdrawing the ascites fluid from the peritoneal cavity with a syringe.

Hybridomas secreting the desired immunoglobulins are cloned and the class and subclass are determined using procedures known in the art. For polyclonal immunoglobulins, immunoglobulin containing antisera is isolated from the immunized animal and is screened for the presence of immunoglobulins with the desired specificity using one of the above-described procedures. The above-described antibodies may also be immobilized on a solid support. Examples of such solid supports include plastics such as polycarbonate, complex carbohydrates such as agarose and sepharose, acrylic resins and such as polyacrylamide and latex beads. Techniques for coupling antibodies to such solid supports are well known in the art.

A plurality of conventional display technologies is available to select an immunoglobulin, immunoglobulin fragment or proteinaceous binding molecule. Li et al. (Organic & Biomolecular Chemistry (2006), 4, 3420-3426) have for example demonstrated how a single-chain Fv fragment capable of forming a complex with a selected DNA adapter can be obtained using phage display. Display techniques for instance allow the generation of engineered immunoglobulins and ligands with high affinities for a selected target molecule. It is thus also possible to display an array of peptides or proteins that differ only slightly, typically by way of genetic engineering. Thereby it is possible to screen and subsequently evolve proteins or peptides in terms of properties of interaction and biophysical parameters. Iterative rounds of mutation and selection can be applied on an *in vitro* basis.

*In vitro* display technology for the selection of peptides and proteins relies on a physical linkage between the peptide or protein and a nucleic acid encoding the same. A large panel of techniques has been established for this purpose, with the most commonly used being phage/virus display, ribosome display, cell-surface display, 'peptides on plasmids', mRNA display, DNA display, and *in vitro* compartmentalisation including micro-bead display (for reviews see e.g. Rothe, A., et al., FASEB J. (2006) 20, 1599 -1610; Sergeeva, A., et al., Advanced Drug Delivery Reviews (2006) 58, 1622-1654).

Different means of physically linking a protein or peptide and a nucleic acid are also available. Expression in a cell with a cell surface molecule, expression as a fusion polypeptide with a viral/phage coat protein, a stabilised *in vitro* complex of an RNA molecule, the ribosome and the respective polypeptide, covalent coupling *in vitro* via a puromycin molecule or via micro-beads are examples of ways of linking the protein/peptide and the nucleic acid presently used in the art. A further display technique relies on a water-in-oil emulsion. The water droplets serve as compartments in each of which a single gene is transcribed and translated (Tawfik, D.S., & Griffiths, A.D., Nature Biotech. (1998) 16, 652-656, US patent application 2007/0105117). This physical linkage between the peptide or protein and the nucleic acid (encoding it) provides the possibility of recovering the nucleic acid encoding the selected protein or peptide. Compared to techniques such as immunoprecipitation, in display techniques thus not only binding partners of a selected target molecule can be identified or selected, but the nucleic acid of this binding partner can be recovered and used for further processing. Present display techniques thus provide means for e.g. target discovery, lead discovery and lead optimisation. Vast libraries of peptides or proteins, e.g. antibodies, potentially can be screened on a large scale.

As indicated above, a detectable marker may be coupled to a binding partner of proSP-B/, effective portion of pro-SP-B, or a molecule that forms a complex with the binding partner of proSP-B. A respective detectable marker, which may be coupled to a binding partner of proSP-B or a molecule that forms a complex therewith, may be an optically detectable label, a fluorophore, or a chromophore. Examples of suitable labels include, but are not limited to, an organic molecule, an enzyme, a radioactive, fluorescent, and/or chromogenic moiety, a luminescent moiety, a hapten, digoxigenin, biotin, a metal complex, a metal and colloidal gold. Accordingly an excitable fluorescent dye, a radioactive label, included for instance in a radioactive amino acid, a fluorescent protein or an enzyme may for instance be used to detect e.g. the level of pro-SP-B. Examples of suitable fluorescent dyes include, but are not limited to, fluorescein isothiocyanate, 5,6-carboxymethyl fluorescein, Cascade Blue^{®}, Oregon Green^{®}, Texas red, nitrobenz-2-oxa-1,3-diazol-4-yl, coumarin, dansyl chloride, rhodamine, amino-methyl coumarin, DAPI, Eosin, Erythrosin, BODIPY^{®}, pyrene, lissamine, xanthene, acridine, an oxazine, phycoerythrin, a Cy dye such as Cy3, Cy3.5, Cy5, Cy5PE, Cy5.5, Cy7, Cy7PE or Cy7APC, an Alexa dye such as Alexa 647, and NBD (Naphthol basic dye). Examples of suitable fluorescent protein include, but are not limited to, EGFP, emerald, EYFP, a phycobiliprotein such as phycoerythrin (PE) or allophycocyanin, Monomeric Red Fluorescent Protein (mRFP), morange, Plum and mCherry. In some embodiments a reversibly photoswitchable fluorescent protein such as Dronpa, bsDronpa and Padron may be employed (Andresen, M., et al., Nature Biotechnology (2008) 26, 9, 1035). Regarding suitable enzymes, alkaline phosphatase, soybean peroxidase, or horseradish peroxidase may serve as a few illustrative examples. Typical radioactive labels include, but are not limited to, ³⁵S, ¹²⁵I, ³²P, and ³³P. A radioactive label can be detected by any method known and appropriate such as a light-sensitive film or a phosphor imager. In some embodiments a method of detection may include electrophoresis, HPLC, flow cytometry, fluorescence correlation spectroscopy or a modified form of these techniques. Some or all of these steps may be part of an automated separation/detection system.

In some embodiments a method of detection may include electrophoresis, HPLC, flow cytometry, fluorescence correlation spectroscopy or a modified form of these techniques. Some or all of these steps may be part of an automated separation/detection system.

Determining the amount of proSP-B, or an effective portion thereof, in the sample can be carried out by way of any suitable technique available. An illustrative example of a suitable technique in this regard is a radiolabel assay such as a Radioimmunoassay (RIA) or an enzyme-immunoassay such as an Enzyme Linked Immunoabsorbent Assay (ELISA), precipitation (particularly immunoprecipitation), a sandwich enzyme immune test, an electrochemiluminescence sandwich immunoassay (ECLIA), a dissociation-enhanced lanthanide fluoro immuno assay (DELFIA), a scintillation proximity assay (SPA), turbidimetry, nephelometry, latex-enhanced turbidimetry or nephelometry, or a solid phase immune test. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described herein. While a RIA is based on the measurement of radioactivity associated with a complex formed between an immunoglobulin or a proteinaceous binding molecule with immunoglobulin-like functions and an antigen, an ELISA is based on the measurement of an enzymatic reaction associated with a complex formed between an immunoglobulin or a proteinaceous binding molecule with immuneglobulin-like functions and an antigen. Typically a radiolabel assay or an enzyme-immunoassay involves one or more separation steps in which a binding partner of e.g. proSP-B that has not formed a complex with proSP-B is being removed, thereby leaving only binding partner of proSP-B behind, which has formed a complex with proSP-B. This allows the generation of specific signals originating from the presence of proSP-B.

An ELISA or RIA test can be competitive for measuring the amount of proSP-B, i.e. the amount of antigen. For example, an enzyme labeled antigen is mixed with a test sample containing antigen, which competes for a limited amount of immunoglobulin or a proteinaceous binding molecule with immunoglobulin-like functions. The reacted (bound) antigen is then separated from the free material, and its enzyme activity is estimated by addition of substrate. An alternative method for antigen measurement is the double immunoglobulin/proteinaceous binding molecule sandwich technique. In this modification a solid phase is coated with specific immunoglobulin or a proteinaceous binding molecule with immunoglobulin-like functions. This is then reacted with the sample from the subject that contains the antigen. Then enzyme labeled specific immunoglobulin/proteinaceous binding molecule is added, followed by the enzyme substrate. The 'antigen' in the test sample is thereby 'captured' and immobilized on to the sensitized solid phase where it can itself then immobilize the enzyme labeled immunoglobulin/proteinaceous binding molecule. This technique is analogous to the immunoradiometric assays.

In an indirect ELISA method, an antigen is immobilized by passive adsorption on to the solid phase. A test serum may then be incubated with the solid phase and any immunoglobulin in the test serum forms a complex with the antigen on the solid phase. Similarly a solution of a proteinaceous binding molecule with immunoglobulin-like functions may be incubated with the solid phase to allow the formation of a complex between the antigen on the solid phase and the proteinaceous binding molecule. After washing to remove unreacted serum components an anti-immunoglobulin immunoglobulin anti-proteinaceous binding molecule immunoglobulin, linked to an enzyme is contacted with the solid phase and incubated. Where the second reagent is selected to be a proteinaceous binding molecule with immunoglobulin-like functions, a respective proteinaceous binding molecule that specifically binds to the proteinaceous binding molecule or the immunoglobulin directed against the antigen is used. A complex of the second proteinaceous binding molecule or immunoglobulin and the first proteinaceous binding molecule or immunoglobulin, bound to the antigen, is formed. Washing again removes unreacted material. In the case of RIA radioactivity signals are being detected. In the case of ELISA the enzyme substrate is added. Its colour change will be a measure of the amount of the immobilized complex involving the antigen, which is proportional to the antibody level in the test sample.

In another embodiment the immunoglobulin or the proteinaceous binding molecule with immunoglobulin-like functions may be immobilized onto a surface, such as the surface of a polymer bead (supra), or coated onto the surface of a device such as a polymer plate or a glass plate. As a result the immune complexes can easily be separated from other components present by simply washing the surface, e.g. the beads or plate. This is the most common method currently used in the art and is referred to as solid phase RIA or ELISA. This embodiment may be particularly useful for determining the amount of proSP-B. On a general basis, in any embodiment of a radiolabel assay or of an enzyme-immunoassay passive adsorption to the solid phase can be used in the first step. Adsorption of other reagents can be prevented by inclusion of wetting agents in all the subsequent washing and incubation steps. It may be advantageous to perform washing to prevent carry-over of reagents from one step to the next.

Various other modifications of ELISA have been used in the art. For example, a system where the second proteinaceous binding molecule or immunoglobulin used in the double antibody sandwich method is from a different species, and this is then reacted with an anti-immunoglobulin enzyme conjugate or an anti-proteinaceous binding molecule enzyme conjugate. This technique comes with the potential advantage that it avoids the labeling of the specific immunoglobulin or proteinaceous binding molecule, which may be in short supply and of low potency. This same technique can be used to assay immunoglobulin or proteinaceous binding molecule where only an impure antigen is available; the specific reactive antigens are selected by the antibody immobilized on the solid phase.

In another example of an ELISA assay for antigen, a surface, a specific antigen is immobilized on a surface, e.g. a plate used, and the surface is then incubated with a mixture of reference immunoglobulins or proteinaceous binding molecules and a test sample. If there is no antigen in the test sample the reference immunoglobulin or proteinaceous binding molecule becomes fixed to an antigen sensitized surface. If there is antigen in the test solution this combines with the reference immunoglobulin or proteinaceous binding molecule, which cannot then react with the sensitized solid phase. The amount of immunoglobulin/proteinaceous binding molecule attached is then indicated by an enzyme labeled anti-globulin/anti-binding molecule conjugate and enzyme substrate. The amount of inhibition of substrate degradation in the test sample (as compared with the reference system) is proportional to the amount of antigen in the test system.

As indicated above, determining the amount of proSP-B, or an effective portion thereof, in the sample typically involves the formation of signals, e.g. signals generated by a detectable marker (supra) that can be quantified. Quantifying the signals in order to determine the amount of proSP-B, or an effective portion thereof, in the sample may be carried out by comparing obtained signals with those of one or more reference measurements. A respective reference measurement is generally based on the signal generated by a known amount of proSP-B. Such a known amount of proSP-B, or an effective portion thereof, may for example be present in a sample with a composition that resembles the sample from the patient, in which the amount of proSP-B, or an effective portion thereof, is to be determined. A respective reference sample may be taken to define an external reference sample. In some embodiments of a method of the invention an internal reference sample may in addition or alternatively be used. Such an internal reference sample is a sample obtained from the subject at a previous point of time. The amount of proSP-B, or an effective portion thereof, in such a sample may be determined to identify the changes in proSP-B levels, or of the/an effective portion thereof, in the subj ect.

In the method of the invention the amount of proSP-B, or an effective portion thereof, determined in the sample is compared to a threshold value. Such a threshold value may in some embodiments be a predetermined threshold value. In some embodiments the threshold value is based the amount of proSP-B, or an effective portion thereof, in a control sample. A respective control sample may have any condition that varies from the sample main measurement itself. Such a control sample may be a sample of, or including, the corresponding body fluid as the sample from the subject or itmay be from amniotic fluid encompassing a respective foetus. A control sample may for example be a sample, such as a blood sample, a plasma sample or a serum sample, of a subject known not to suffer from obstructive pulmonary disease. A control sample may also be from a newborn subject known not to suffer from a respiratory disorder, or it may be amniotic fluid encompassing a foetus known after birth not to suffer from a respiratory disorder. In some embodiments a respective control sample is from a subject that is age-matched. In some embodiments a respective control sample is from a subject that is known not to have a confounding disease, in some embodiments from a subject known not to have a disease.

In some embodiments a threshold value is a collection of data of a plurality of control samples, which may also be referred to as a reference samples. In such embodiments the threshold value may be set to be a significant difference between the control and the sample from the subject of interest. The term "significant" is used to indicate that the level of increase is of statistical relevance. As an illustrative example a plurality of measurements, including a plurality of samples may have been obtained from the subject of interest. The p value may then be determined. A p value of 0.05, 0.02, 0.01 or lower may be taken to indicate a difference. In some embodiments a significant increase is a deviation of a value of a test sample relative to a value of a control sample of about 2 fold or more, including 3 fold or more, such as at least about 5 to about 10 fold or even more.

In some embodiments a threshold value is based on a control or reference value obtained concomitantly with the value of the sample from the subject. In some embodiments a respective control or reference value is determined at a different point in time, for example at a point in time earlier than the measurement of the sample from the subject is carried out. It is understood that the terms control and reference may in some embodiments be a range of values.

The comparison to a threshold value, which may be a predetermined threshold value, can be carried out manually, semi-automatically or in a fully automated manner. In some embodiments the comparison may be computer assisted. A computer assisted comparison may employ values stored in a database as a reference for comparing an obtained value or a determined amount, for example via a computer implemented algorithm. Likewise, a comparison to a reference measurement may be carried out manually, semi-automatically or in a fully automated manner, including in a computer assisted manner.

A predetermined threshold value may in some embodiments be set on the basis of data collected from one or more newborns or foetuses known not to suffer from a respiratory disorder. In some embodiments a certain percentile of such data may be used as a threshold value. The range of the values of a set of data obtained from such patients can be divided into 100 equal parts, i.e. percentages of the range can be determined. A percentile represents the value within the respective range below which a certain percent of the data fall, in other words the percentage of the values that are smaller than that value. For example the 95th percentile is the value below which 95 percent of the data are found. In some embodiments a level of proSP-B, or an effective portion thereof, may be regarded as increased or elevated if it is above the 90^{th} percentile, above the 92^{nd} percentile, above the 93^{rd} percentile, above the 94^{th} percentile, above the 95^{th} percentile, above the 96^{th} percentile, above the 97^{th} percentile, above the 98^{th} percentile or above the 99^{th} percentile.

In some embodiments the average value of data from newborn or foetuses known not to suffer from a respiratory disorder may be determined and used to determine a threshold value. As an illustrative example, the value of standard deviation or relative standard deviation, in some embodiments multiplied with a factor, which may be larger or smaller than 1. In some embodiments a multiple of standard deviation or relative standard deviation, such as 1.2, 1.5 or 2 times the standard deviation, may be added to the average value determined. In some embodiments establishing a predetermined threshold value includes collecting from newborn or foetuses known not to suffer from a respiratory disorder over a period of time, such as a plurality of weeks. Average proSP-B levels, or levels of an effective portion thereof, standard deviation, and relative standard deviation at given times may be calculated for the subjects to determine a range of proSP-B levels associated with absence of respiratory disorder or with a healthy condition. In some embodiments an average of a selected number, such as 2, 3, 4, 5 or more of the highest proSP-B levels determined over time may be used as a predetermined threshold value. In some embodiments the average value of all data collected at different time points may be determined and used to determine a threshold value, for instance by adding standard deviation, relative standard deviation or e.g. a multiple thereof (supra). When test result from a subject to be evaluated is collected, it will be compared to the threshold value. Statistical differences between the test result and the threshold value will be determined to identify significant variances in between.

The amount of proSP-B, or an effective portion thereof, determined in or from a sample of a subject can be compared to a single control sample or a plurality of control samples, such as a sample from a control subject, in any suitable manner. As an illustrative example, the level of proSP-B, or an effective portion thereof, in a control sample can be characterized by an average (mean) value coupled with a standard deviation value, for example at a given time point. In some embodiments the level of proSP-B, or an effective portion thereof, in a subject may be considered increased when it is one standard deviation or more higher than the average value of the corresponding expression level determined in one or more control samples. In some embodiments the determined level of proSP-B, or an effective portion thereof, is regarded as increased where the obtained value is about 1.5 standard deviations higher or lower, including about two, about three, about four or more standard deviations higher or lower than the average value determined in a control sample. In some embodiments the determined amount of proSP-B, or an effective portion thereof, is regarded as different where the obtained value is about 1.2 times or more higher or lower, including about 1.5 times, about two fold, about 2.5-fold, about three fold, about 3.5 fold, about 4-fold, about 5-fold or more higher or lower than the protein level determined in a control sample. In some embodiments the determined level of proSP-B, or an effective portion thereof, is regarded as increased where the obtained value is about 0.8-fold or less, including about 70 %, about 60 %, about 50 %, about 40 %, about 30 %, about 25 %, about 20 % or lower than the of proSP-B level, or level of an effective portion thereof, determined in a control sample.

If for example a level of proSP-B, or an effective portion thereof, is detected that is above a, e.g. predetermined, threshold value, this may indicate the occurrence or the risk of occurrence of a respiratory disorder, in particular respiratory distress syndrome, in a newborn. A level of proSP-B, or an effective portion thereof, above a threshold value may indicate a condition where the subject is in need of respiratory support therapy. If a level of proSP-B, or an effective portion thereof, is detected that is below a predetermined threshold value, this may indicate that no respiratory disorder has occurred, as well as that the risk of occurrence of a respiratory disorder is low.

In some embodiments the amount of proSP-B, or an effective portion thereof, in a sample is being detected repeatedly. In some embodiments repeated detection is carried out on different samples from the same individual. In some embodiments a plurality of measurements is carried out on a plurality of samples from the same patient. In each of the samples the level of proSP-B, or an effective portion thereof, is determined. Typically the level of proSP-B, or an effective portion thereof, determined in each of the samples is compared to a threshold value as detailed above. In some embodiments the plurality of samples from the same individual is taken over a period of time at certain time intervals, including at predetermined time intervals. Such an embodiment may be taken as a method of monitoring the amount of proSP-B, or an effective portion of proSP-B. As an illustrative example, samples may be taken from the same subject at a time interval of about 3 days. In some embodiments samples may be taken from the same subject at a time interval of about 2 days. In some embodiments samples may be taken from the same subject at a time interval of about one day, e.g. once per day, including about every 24 hours. In some embodiments samples may be taken from the same subject at a time interval of about 18 hours. In some embodiments samples may be taken from the same subject at a time interval of about 12 hours or of about 8 hours. A value obtained from a respective sample may in some embodiments be compared to a sample taken from the same subject at a previous point of time, for example on the previous day. In this way a change in the level of proSP-B, or the level of a respective effective portion of proSP-B, may be detected. Matching samples may in some embodiments be used to determine a threshold value for each corresponding time point. The average value may be determined and the standard deviation calculated for each given time point. A value determined in the sample from the subject falling outside of the mean plus 1 standard deviation may for instance be indicative of the occurrence or of the risk of occurrence of a respiratory disorder.

Monitoring the amount of proSP-B, or an effective portion thereof, may be included in the context of monitoring a therapy, for example in order to assess the efficacy thereof or to evaluate a subject's response to a certain treatment.

In some embodiments the new born subject has been diagnosed not to suffer from an infection. In embodiments where a newborn does not have an infection, and amniotic fluid encompassing the respective foetus is or has been taken, a suitable threshold value in such amniotic fluid may be inferred from Fig. 4 to Fig. 7. A respective threshold value may be applied in any method and/or use disclosed herein, such as a method of determining whether a new born subject is in need of respiratory support therapy, a method of determining whether a foetus will be in need of respiratory support therapy after birth, a method of stratifying a new born subject or a foetus for respiratory support therapy, a method of determining the occurrence of a respiratory disorder in a new born subject or a foetus, a method of assessing the risk of occurrence of a respiratory disorder in a new born subject or a foetus, a method of stratifying a new born subject or a foetus for risk of occurrence of a respiratory disorder, the use of a binding agent for determining whether a new born subject is in need of respiratory support therapy, the use of a binding agent for determining whether a foetus will be in need of respiratory support therapy after birth and the use of a binding agent for stratifying a new born subject or a foetus for respiratory support therapy.

A suitable threshold value in any such method or use may be a value of above about 75 ng/ml proSP-B, including above about 90 ng/ml, which may indicate the need of respiratory support therapy, including a risk of occurrence of a respiratory disorder. A suitable threshold value may also be a level above about 100 ng/ml, such as above about 110 ng/ml or above about 120 ng/ml proSP-B. In some embodiments a suitable threshold value is a value above about 130 ng/ml, including above about 140 ng/ml or above about 150 ng/ml proSP-B. A suitable threshold value may further be a level above about 160 ng/ml proSP-B. A suitable threshold value may in some embodiments be a level above about 170 ng/ml, including above about 180 ng/ml proSP-B. A suitable threshold value, which may indicate the need of respiratory support therapy, including a risk of occurrence of a respiratory disorder, may also be a level above about 190 ng/ml proSP-B. In some embodiments a suitable threshold value may be a level above about 200 ng/ml. In some embodiments a suitable threshold value may also be a value above about 210 ng/ml, including above about 220 ng/ml proSP-B. A suitable threshold value may also be a level above about 230 ng/ml proSP-B. In some embodiments a suitable threshold value may be a value above about 250 ng/ml proSP-B. A suitable threshold value may also be a level above about 270 ng/ml proSP-B. A suitable threshold value may furthermore be a level above about 300 ng/ml proSP-B. In some embodiments a suitable threshold value may also be a level above about 320 ng/ml, such as above about 350 ng/ml proSP-B. A suitable threshold value may also be a level above about 380 ng/ml proSP-B. A suitable threshold value may also be a level above about 400 ng/ml or above about 450 ng/ml proSP-B, which may indicate the need of respiratory support therapy, including a risk of occurrence of a respiratory disorder. A value above about 300 ng/ml, including above about 350 ng/ml of the respective effective portion of proSP-B, for instance a peptide defined by or that is corresponding to the amino acid positions 285 to 334 of the sequence of Swissprot/Uniprot accession number P07988 (supra, SEQ ID NO: 9), may indicate the need of respiratory support therapy, including a risk of occurrence of a respiratory disorder. In this regard a value above about 400 ng/ml of the corresponding effective portion of proSP-B may be a suitable threshold value. In some embodiments a suitable threshold value in this regard is a value above about 450 ng/ml, such as above about 480 ng/ml of the respective effective portion of proSP-B, e.g. the portion corresponding to the amino acid positions 285 to 334 of the sequence of Swissprot/Uniprot accession number P07988. In some embodiments a suitable threshold value in this regard is a value above about 500 ng/ml of the respective effective portion of proSP-B, including above about 520 ng/ml of the respective effective portion of proSP-B. A suitable threshold value may also be a level above about 540 ng/ml, such as above about 560 ng/ml of the respective effective portion of proSP-B. In some embodiments a suitable threshold value is a value above about 580 ng/ml of the respective effective portion of proSP-B. In some embodiments a suitable threshold value is a value above about 600 ng/ml of the respective effective portion of proSP-B of the respective effective portion of proSP-B of the respective effective portion of proSP-B. In some embodiments a suitable threshold value is a value above about 620 ng/ml of the respective effective portion of proSP-B. A suitable threshold value is in some embodiments a value above about 640 ng/ml of the respective effective portion of proSP-B. A value above about 660 ng/ml, such as above about 680 ng/ml of the respective effective portion of proSP-B is in some embodiments a respective suitable threshold value. In some embodiments a suitable threshold value is a value above about 700 ng/ml of the respective effective portion of proSP-B, e.g. the portion corresponding to the amino acid positions 285 to 334 of the sequence of Swissprot/Uniprot accession number P07988. In some embodiments a suitable threshold value is a value above about 720 ng/ml, including above about 740 ng/ml of the respective effective portion of proSP-B. In some embodiments a value of above about 760 ng/ml, including above about 780 ng/ml of the respective effective portion of proSP-B is a respective suitable threshold value. In some embodiments a suitable threshold value in this regard is a value above about 800 ng/ml of the respective effective portion of proSP-B may be a suitable threshold value. A value above about 850 ng/ml of the respective effective portion of proSP-B may in some embodiments be a suitable threshold value. A suitable threshold value is in some embodiments a value above about 900 ng/ml of the respective effective portion of proSP-B, e.g. the portion corresponding to the amino acid positions 285 to 334 of the sequence of Swissprot/Uniprot accession number P07988 (supra, SEQ ID NO: 9). In some embodiments a suitable threshold value is a value above about 950 ng/ml of the respective effective portion of proSP-B. In some embodiments a suitable threshold value is a value above about 1000 ng/ml, such as above about 1050 ng/ml of the respective effective portion of proSP-B. In some embodiments a value above about 1100 ng/ml, above about 1150 ng/ml or above about 1200 ng/ml of the respective effective portion of proSP-B, for instance a peptide defined by or that is corresponding to the amino acid positions 285 to 334 of the sequence of Swissprot/Uniprot accession number P07988, may indicate the need of respiratory support therapy, including a risk of occurrence of a respiratory disorder.

As can be taken from Fig. 3 to Fig. 7, subjects that were assessed by physicians as being in need of respiratory support therapy, i.e. using established diagnosis methods, were generally subjects for whom amniotic fluid had shown increased levels of pro-SpB, and/or of the C-terminal fragment thereof. As shown in Fig. 1 and Fig. 2, a predetermined threshold value may be selected based on the analysis of existing data. A suitable threshold value for a particular case may be determined on the basis of the desired information the assessment shall provide. If high reliability in terms of whether a subject may require respiratory support therapy is desired, it may be useful to determine sensitivity of existing data. Sensitivity indicates the portion of the test results that correctly indicated that a subject would be in need of respiratory support therapy, thereby reflecting the probability to have a correct prediction of respiratory support therapy requirement for a subject. Accordingly, in this case a threshold value is selected that results in a high portion of data above the threshold value that correspond to subjects assessed as being in need of respiratory support therapy by standard methods. While a lower threshold value increases the likelihood of correctly predicting that a subject will be in need of respiratory support therapy, it will include "false positives", i.e. a portion of subjects not requiring respiratory support therapy, being subjects with per se relatively high levels of proSP-B. Thus Fig. 1 shows (cf. curve 1) an increasing reliability of including most, up to all, subject requiring respiratory support therapy with a decreasing threshold value. Accordingly, in any case a balance will have to be struck between (a) a high precision of prediction and maintaining a range into which a desired portion, such as the majority of subject in need of respiratory support therapy, fall and (b) a manageable and reasonable low number of false predictions of a need for respiratory support therapy for subjects who in fact do not need respiratory support therapy.

In some embodiments a suitable threshold value in this regard may be a value of above about 600 ng/ml proSP-B. In some embodiments a suitable threshold value in this regard may be a value of above about 557 ng/ml, including above about 550 ng/ml proSP-B. In some embodiments a suitable threshold value in this regard may be a value of above about 510 ng/ml, including above about 450 ng/ml or above about 400 ng/ml proSP-B. In some embodiments a suitable threshold value in this regard may be a value of above about 350 ng/ml proSP-B. In some embodiments a suitable threshold value in this regard may be a value of above about 325 ng/ml, including above about 275 ng/ml proSP-B. In some embodiments a suitable threshold value may be a value of above about 250 ng/ml, such as above about 200 ng/ml or above about 175 ng/ml proSP-B.

In some embodiments a suitable threshold value in this regard may be a value of above about 1420 ng/ml, such as above about 1380 ng/ml of an effective portion of proSP-B, such as the C-terminal fragment of proSP-B. In some embodiments a suitable threshold value in this regard may be a value of above about 1300 ng/ml, including above about 1250 ng/ml or 1210 ng/ml of an effective portion of proSP-B, such as the C-terminal fragment of proSP-B. In some embodiments a suitable threshold value in this regard may be a value of above about 1180 ng/ml, including above about 1150 ng/ml or above about 1130 ng/ml of an effective portion of proSP-B, such as the C-terminal fragment of proSP-B. In some embodiments a suitable threshold value in this regard may be a value of above about 1100 ng/ml including above about 1050 ng/ml of an effective portion of proSP-B, such as the C-terminal fragment of proSP-B. In some embodiments a suitable threshold value in this regard may be a value of above about 1000 ng/ml, including above about 975 ng/ml of an effective portion of proSP-B, such as the C-terminal fragment of proSP-B. In some embodiments a suitable threshold value may be a value of above about 950 ng/ml, such as above about 900 ng/ml or above about 850 ng/ml of an effective portion of proSP-B, such as the C-terminal fragment of proSP-B. In some embodiments a suitable threshold value in this regard may be a value of above about 800 ng/ml, including above about 750 ng/ml or above about 680 ng/ml of an effective portion of proSP-B, such as the C-terminal fragment of proSP-B. In some embodiments a suitable threshold value in this regard may be a value of above about 650 ng/ml, including above about 600 ng/ml, above about 500 ng/ml or above about 475 ng/ml of an effective portion of proSP-B, such as the C-terminal fragment of proSP-B.

If high reliability in terms of whether a subject will be healthy in that it will not require respiratory support therapy is desired, it may be useful to determine specificity of existing data. Sensitivity indicates the portion of the test results that correctly indicated that a subject would not be in need of respiratory support therapy, thereby reflecting the probability to have a correct prediction for a healthy subject that no respiratory support therapy will be necessary. Accordingly, in this case a threshold value is selected that results in a high portion of data below and up to the threshold value that correspond to subjects assessed as being healthy, i.e. not in need of respiratory support therapy by standard methods. While a higher threshold value increases the likelihood of correctly predicting that a subject will not be in need of respiratory support therapy, and thus conversely allow predicting or excluding healthy subjects, it will miss a portion of subjects in need of respiratory, being subjects with levels of proSP-B that are increased to relatively moderately amounts. Accordingly, in any case a balance will have to be struck between a high precision of prediction and maintaining a range into which a desired portion, such as the majority of healthy subjects fall.

A suitable threshold value in this regard may in some embodiments be a value of above about 400 ng/ml proSP-B. In some embodiments a suitable threshold value in this regard may be a value of above about 375 ng/ml, including above about 350 ng/ml proSP-B. In some embodiments a suitable threshold value in this regard may be a value of above about 320 ng/ml, including above about 290 ng/ml or above about 275 ng/ml proSP-B. In some embodiments a suitable threshold value in this regard may be a value of above about 250 ng/ml proSP-B. In some embodiments a suitable threshold value in this regard may be a value of above about 225 ng/ml, including above about 200 ng/ml proSP-B. In some embodiments a suitable threshold value may be a value of above about 180 ng/ml, such as above about 140 ng/ml or above about 100 ng/ml proSP-B.

In some embodiments a suitable threshold value in this regard may be a value of above about 1100 ng/ml, such as above about 1050 ng/ml of an effective portion of proSP-B, such as the C-terminal fragment of proSP-B. In some embodiments a suitable threshold value in this regard may be a value of above about 850 ng/ml, including above about 800 ng/ml of an effective portion of proSP-B, such as the C-terminal fragment of proSP-B. In some embodiments a suitable threshold value in this regard may be a value of above about 780 ng/ml, including above about 720 ng/ml of the C-terminal fragment of an effective portion of proSP-B, such as proSP-B. A suitable threshold value in this regard may in some embodiments be a value of above about 690 ng/ml, such as above about 670 ng/ml of an effective portion of proSP-B, such as the C-terminal fragment of proSP-B. In some embodiments a suitable threshold value in this regard may be a value of above about 620 ng/ml, including above about 570 ng/ml of an effective portion of proSP-B, such as the C-terminal fragment of proSP-B. In some embodiments a respectively suitable threshold value may be a value of above about 540 ng/ml, including above about 520 ng/ml of an effective portion of proSP-B, such as the C-terminal fragment of proSP-B. In some embodiments a suitable threshold value in this regard may be a value of above about 500 ng/ml, including above about 470 ng/ml of an effective portion of proSP-B, such as the C-terminal fragment of proSP-B.

In some embodiments it may be desirable to base a threshold value on the ratio of sensitivity and specificity (supra), i.e. the mean of sensitivity and specificity, as illustrated in Fig. 1 and Fig. 2. A suitable threshold value in this regard may in some embodiments be a value of above about 570 ng/ml proSP-B. In some embodiments a suitable threshold value in this regard may be a value of above about 540 ng/ml proSP-B. In some embodiments a suitable threshold value in this regard may be a value of above about 400 ng/ml, including above about 440 ng/ml proSP-B. A suitable threshold value in this regard may in some embodiments be a value of above about 265 ng/ml proSP-B. In some embodiments a suitable threshold value in this regard may be a value of above about 180 ng/ml proSP-B.

In some embodiments a suitable threshold value in this regard may be a value of above about 1130 ng/ml of an effective portion of proSP-B, such as the C-terminal fragment of proSP-B. In some embodiments a suitable threshold value in this regard may be a value of above about 1030 ng/ml, such as above about 1050 ng/ml of an effective portion of proSP-B, such as the C-terminal fragment of proSP-B. In some embodiments a suitable threshold value in this regard may be a value of above about 710 ng/ml, including above about 775 ng/ml of an effective portion of proSP-B, such as the C-terminal fragment of proSP-B. In some embodiments a suitable threshold value in this regard may be a value of above about 550 ng/ml of an effective portion of proSP-B, such as the C-terminal fragment of proSP-B.

In cases where the newborn has an infection that is not related to lung function, e.g. a skin infection, an ear infection, herpes, Toxoplasma, rubella, CMV or hepatitis, and the newborn does not suffer from a lung injury the above threshold values generally apply mutatis mutandis to a corresponding amniotic fluid sample. Where the newborn has a lung injury, including an impairment of lung function due to e.g. systemic inflammation, a threshold value is advantageously based on levels of proSP-B or an effective portion thereof, in one or more corresponding samples, e.g. amniotic fluid, taken from one or more subjects suffering from the same impairment or disease. An increased amount of proSP-B, or an effective portion thereof, relative to a corresponding threshold value, indicates that the subject is suffering from a respiratory disorder and/or in need of respiratory support therapy and vice versa.

In some embodiments a method of the invention is a method of stratifying a subject for respiratory support therapy. The terms "stratifying" and "stratification" as used herein indicate in one aspect that individuals are assigned to groups with similar characteristics such as at a level of obstructive pulmonary disease. As an illustrative example, individuals may be stratified into risk categories, for example whether the individual is or is not at a disease state where potential life threatening changes are to be taken into account. The terms "stratifying" and "stratification" as used herein indicate in another aspect that an individual is assigned to a certain group according to characteristics matching the respective group such as a corresponding level, including risk level of obstructive pulmonary disease. The groups may be, for example, for testing, prescribing, suspending or abandoning any one or more of a drug, surgery, diet, exercise, or intervention. Accordingly, in some embodiments of a method or use according to the invention a subject may be stratified into a subgroup of a clinical trial of a therapy.

The terms "stratifying" and "stratification" according to the invention generally include identifying subjects that require an alteration of their current or future therapy. The term includes assessing, e.g. determining, which therapy a subject suffering from a respiratory disorder is in need of. Hence, in the context of the present invention stratification may be based on the health risk involved with a subject's state of a respiratory disorder. A method or use according to the invention may also serve in stratifying the risk of a respiratory disorder related condition for a subject. As will be apparent from the above, terms "stratifying" and "stratification" include the staging of subjects. The stage of a respiratory disorder in terms of disease severity can be assessed using a method according to the invention. A method of stratifying a subject for a respiratory disorder therapy according to the invention includes detecting the amount of pro-SP-B, or an effective portion thereof, as described above.

In this regard the use of biomarkers for stratification of patients is a well established procedure in the art. This procedure includes or consists of linking one or more patient subpopulations, characterized by a certain feature, in the context of the present invention the level of particular proteins, to a particular treatment. The general aim of stratification is to match patients with therapies that are more likely to be effective and safe. In a more general context stratifying patients may include evaluation of patient history and physical assessment, combined with laboratory tests on the basis of a method of the present invention, and clinical observation. It is understood that stratifying patients is only feasible if multiple treatment options with heterogeneous responses for the disease exist. In the context of the present invention therapy of obstructive pulmonary disease and patient surveillance may be adjusted. A general overview of patient stratification and stratified medicine has been given by Trusheim, M.R., et al., Nature Reviews Drug Discovery (2007) 6, 4, 287-293.

The present invention also relates to a combination of a binding agent and a predetermined amount of at least one of proSP-B and the effective portion of proSP-B. As explained above in more detail, the binding agent may be an immunoglobulin or a proteinaceous binding molecule with immunoglobulin-like functions, with the binding molecule or the immunoglobulin, respectively, being specific for proSP-B. The effective portion of proSP-B may for example include or be a C-terminal portion of proSP-B, such as the portion corresponding to the amino acid positions 285 to 334 of the sequence of Swissprot/Uniprot accession number P07988 (supra, SEQ ID NO: 9). The predetermined amount of proSP-B and the predetermined amount of the effective portion of proSP-B can be used to provide a reference in the form of a known amount of proSP-B and the effective portion of proSP-B, respectively. A series of concentrations may for example be prepared that can be used to create a standard curve of the corresponding peptide/protein.

In some embodiments the combination is provided in the form of a kit. The kit may include a first container that includes the binding molecule, e.g. the immunoglobulin or the proteinaceous binding molecule with immunoglobulin-like functions specific for proSP-B. The kit may further include a second container that includes the predetermined amount of proSP-B and/or the predetermined amount of the effective portion of proSP-B. The kit may include further reagents needed or useful in the context of the present invention.

In order that the invention may be readily understood and put into practical effect, particular embodiments will now be described by way of the following non-limiting examples.

### EXAMPLES

### EXAMPLES FOR MEASUREMENT OF proSP-B ASSAY

### ANTIBODIES USED

The pro SP-B assay uses a mouse monoclonal anti-proSP-B (N-terminus) antibody as a capture and a mouse monoclonal anti-proSP-B (C-terminus) antibody as a detection reagent. The antibody was generated using established standard techniques. The assay principle is a sandwich format. The antibody to the N-terminal pro-sequence (clone 1.14.133) binds to an epitope comprised in the peptide sequence ranging from amino acid 160 to 169 of proSP-B (SEQ ID NO: 6). The antibody to the C-terminal pro-sequence (clone 1.7.41) binds to an epitope comprised in the peptide sequence ranging from amino acid 323 to 334 of proSP-B (SEQ ID NO: 7). Detection is based on an electrochemiluminescence immunoassay (ECLIA), using a Tris(bipyridyl)-ruthenium(II) complex as a label.

### ASSAY FORMAT AND COMPONENTS OF proSP-B ASSAY

A biotinylated capture antibody (80 µl), the ruthenium-labeled detection antibody (80 µl), and sample or standard material (10 µl) are incubated in homogeneous phase for 9 min at 37°C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for proSP-B is given in ng/ml. Data shown in Figures 1, 3, 5 and 7 are based on the use of this assay on different samples as indicated.

### EXAMPLES FOR MEASUREMENT OF C-TERMINAL FRAGMENT OF proSP-B

The C-fragment pro SP-B assay uses mouse monoclonal anti-proSP-B (C-terminus) antibodies for capture and another mouse monoclonal anti proSP-B (C-terminus) antibodie as a detection reagent. The assay principle is a sandwich format. Detection is either based on an electrochemiluminescence immunoassay (ECLIA), or a POC assay format.

The C-fragment pro SP-B assay uses a first mouse monoclonal anti-proSP-B (C-terminus) antibody as a capture and a second mouse monoclonal anti proSP-B (C-terminus) antibody as a detection reagent. The assay principle is a sandwich format. The antibody to the first C-terminal pro-sequence (clone 1.7.41) binds to an epitope comprised in the peptide sequence ranging from amino acid 323 to 334 of proSP-B. The antibody to the second C-terminal pro-sequence (clone 1.3.9) binds to an epitope comprised in the peptide sequence ranging from amino acid 285 to 294 of proSP-B (SEQ ID NO: 8). Detection is based on an electrochemiluminescence immunoassay (ECLIA), using a Tris(bipyridyl)-ruthenium(II) complex as label.

### ASSAY PROCEDURE

The biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for C-terminal proSP-B is given in ng/ml. Data shown in Figures 2, 3, 4 and 6 are based on the use of this assay on different samples as indicated.

The invention illustratively described herein may suitably be practiced in the absence of any element or elements, limitation or limitations, not specifically disclosed herein. Thus, for example, the terms "comprising", "including," containing", etc. shall be read expansively and without limitation. Singular forms such as "a", "an" or "the" include plural references unless the context clearly indicates otherwise. Unless otherwise indicated, the term "at least" preceding a series of elements is to be understood to refer to every element in the series. The terms "at least one" and "at least one of" include for example, one, two, three, four, or five or more elements. Slight variations above and below the stated ranges can be used to achieve substantially the same results as values within the ranges. Also, unless indicated otherwise, the disclosure of the ranges is intended as a continuous range including every value between the minimum and maximum values.

Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. A method of (i) determining whether a new born subject is in need of respiratory support therapy, (ii) determining whether a foetus will be in need of respiratory support therapy after birth or (iii) stratifying a new born subject or a foetus for respiratory support therapy, the method comprising detecting the amount of pro-Pulmonary surfactant-associated protein B (proSP-B), or an effective portion thereof, in a sample from the new born subject or foetus, or from amniotic fluid encompassing the foetus, wherein an increased amount of proSP-B, or the portion thereof, relative to a threshold value, indicates a need of the new born subject or the foetus after birth for respiratory support therapy.

2. The method of claim 1, wherein the method is a method of at least one of detecting, predicting and stratifying respiratory distress syndrome in the new born subject or the foetus.

3. The method of claims 1 or 2, wherein the threshold value is based on the amount of proSP-B in a control sample.

4. The method of any one of claims 1 to 3, comprising stratifying the new born subject or the foetus for respiratory support therapy if the amount of proSP-B, or the portion thereof, relative to the threshold value is increased, and not stratifying the new born subject or the foetus for respiratory support therapy if the amount of proSP-B, or the portion thereof, relative to a threshold value is not increased or below the threshold value

5. The method of any one of claims 1 to 4, wherein the new born subject has been diagnosed not to suffer from an infection.

6. The method of any one of claims 1 to 5, wherein the sample is amniotic fluid and wherein the amniotic fluid has been obtained from gestation week 23 to gestation week 41, or later.

7. The method of any one of claims 1 to 6, wherein the sample comprises one of cord blood and serum.

8. The method of any one of claims 1 to 7, wherein proSP-B has the amino acid sequence of SEQ ID NO: 1 and/or wherein the portion of proSP-B comprises the amino acid sequence of SEQ ID NO: 5 or the amino acid sequence of SEQ ID NO: 8.

9. The method of any one of claims 1 to 8, wherein the sample is amniotic fluid (a) of a human foetus, or (b) of a new born human, the sample having been taken before birth, and
wherein (i) the threshold value of the proSP-B is about 265 ng/ml and/or (ii) the threshold value of the portion of proSP-B defined by the amino acid sequence of SEQ ID NO: 5 is about 775 ng/ml.

10. The method of any one of claims 1 to 9, wherein respiratory support therapy comprises mechanical ventilation or non-invasive ventilation.

11. The method of any one of claims 1 to 10, comprising repeatedly detecting the amount of proSP-B, or an effective portion thereof, in a sample from the subject.

12. The method of any one of claims 1 to 11, wherein detecting the amount ofproSP-B, or a portion thereof, comprises contacting the sample with a binding agent, the binding agent being specific for proSP-B, or the portion thereof, and detecting the amount of the binding agent binding to proSP-B.

13. The *in-vitro* use of a binding agent specific for proSP-B, or an effective portion thereof, for (i) determining whether a new born subject is in need of respiratory support therapy, (ii) determining whether a foetus will be in need of respiratory support therapy after birth or (iii) stratifying a new born subject or a foetus for respiratory support therapy.

14. The use of claim 13, comprising the detection of the amount of proSP-B, or a portion thereof, in the sample by means of the binding agent.

15. A kit of parts for (i) determining whether a new born subject is in need of respiratory support therapy, (ii) determining whether a foetus will be in need of respiratory support therapy after birth or (iii) stratifying a new born subject or a foetus for respiratory support therapy, the kit comprising:
a binding agent, the binding agent being specific for proSP-B, or an effective portion thereof, and
a predetermined amount of at least one of proSP-B and the effective portion thereof.
